# EUROPEAN PATENT APPLICATION

(11) **EP 2 481 422 A2**
(43) Date of publication of application: **01.08.2012**
(21) Application number: 11161571.2
(22) Date of filing: 01.09.2004
(51) Int. Cl.: A61K 39/385, G01N 33/53, G01N 33/567

(54) **Multiplex vaccines**

(30) Priority: 03.09.2003 US 500216 P
(62) Divisional of application: 04809655.6
(71) Applicant: DENDRITHERAPEUTICS, INC., Danville, CA 94506 (US)
(72) Inventor: Maida III, Anthony E., Danville California 94506 (US)
(74) Representative: Read, David Graham

(57) **Abstract**

The present invention provides antigen complexes comprising 15 or more, in some instances 15 to 100 or more, different antigens and/or compositions comprising the antigen complexes where the composition comprises 15 or more, in some instances 15 to 100 or more, different antigens, The invention also provides to methods of modulating immune responses through administration of the complexes to an individual and to methods of identifying immunodominant epitopes with use of the antigen complexes.

## Description

### TECHNICAL FIELD

The present invention generally relates to antigen complexes useful in modulating an immune response. More particularly, the invention relates to antigen complexes comprising 15 or more, in some instances 15 to 100 or more, different antigens and/or compositions comprising the antigen complexes where the composition comprises 15 or more, in some instances 15 to 100 or more, different antigens. The invention also relates to methods of modulating immune responses through administration of the complexes to an individual and to methods of identifying immunodominant epitopes with use of the antigen complexes.

### BACKGROUND OF THE INVENTION

T cells mediate many responses, including those generally responsible for clearance of intracellular pathogens, virus-infected cells, tumor cells, as well as those responsible for transplant rejection and autoimmunity. The T cell immune system is adapted to recognizing altered self-cells and eliminating them from the body. In autoimmune diseases, self-tolerance is lost and the immune system attacks "self" tissue as if it were a foreign target.

T cell recognition of peptide antigens occurs via the T cell receptor (TCR) and requires that such antigen be presented to the TCR by a major histocompatibility complex (MHC) molecule situated, for example, on the surface of an antigen presenting cell (APC). The MHC molecules of human are referred to as human histocompatibility leukocyte antigens (HLA) and murine MHC molecules are referred to as H2 molecules. The peptide antigen is held by the MHC molecule such that the T cell receptor recognizes the unique structure formed by the combination of the MHC molecule and the specific peptide, Polymorphisms in the MAC molecules, as well as the wide spectrum of unique peptides that can associate with the MHC, result in an extremely diverse recognition pattern such that a given MHC-peptide combination is only recognized by a small percentage of T cell clones.

There are two primary types of MHC molecules involved in antigen presentation: class I and class II. MHC class I molecules are composed of an alpha chain with 3 domains (α1, α2, and α3), as well as transmembrane and cytoplasmic domains. The α1 and α2 domains are polymorphic. A non-polymorpmc protein, β2-microglobulin, self associates with the alpha chain and is necessary for stable conformation. MHC class I molecules are widely distributed and are present on all nucleated cells. MHC class II molecules are composed of an alpha chain and a beta chain that self associate to form a heterodimer. Each chain has two extracellular domains (α1, α2 and β1, β2), as well as transmembrane and intracellular domains. The α1 and β1 domains are polymorphic. MHC class II molecules are more restricted in distribution than are class I molecules and are present, for example, on APCs.

Cytotoxic T lymphocytes ("CTL") which have been specifically activated against a particular antigen are capable of killing the cell that contains or expresses the antigen. The TCR of a CTL recognizes an antigen in the context of a MHC class I molecule. An important role for T helper lymphocytes ("Th cells") is the optimal induction of a CTL response and they may also play a role in maintenance of CTL memory. The TCR of a Th cell recognizes an antigen in the context of a MHC class II molecule.

Present methods for modulating T cell function suffer from a number of limitations including lack of specificity. For example, therapies for enhancing T cell function (such as in certain infections and malignancies) are often insufficient to induce an adequate immune response. Immunization with peptides alone has often not been successful at inducing a clinically sufficient T cell response.

Multivalent vaccines combining two or more antigenic peptides have been described in the art. For example, a multiple antigen peptide system involving a T cell epitope and a B cell epitope attached to a dendritic core is described in U.S. Pat. Nos. 5,229,490 and 5,580,563 and in PCT Publication No. WO 90/11778. The combination of a CTL epitope and a Th epitope has resulted in induction of protective CTL-mediated immunity. See, for example, Ossendorp et al. (1998) J. Exp. Med. 187:693-702. Multivalent vaccines known in the art, however, generally include only a few different antigens and so rely on the immune response generated to the few particular antigens to provide effective treatment to the individual in need of treatment.

Also, therapies for suppressing T cell function (such as in autoimmunity or for preventing transplant rejection) often cause generalized immunosuppression and may leave patients at risk for developing life-threatening infections. The ultimate goal of anti-T cell immunosuppressive therapy is to inhibit specific T cell alloreactive or autoreactive clones while leaving the majority ofT cells fully functional. Specific immunosuppressive therapy requires targeting T cell clones recognizing specific MHC/peptide combinations, Several researchers have attempted to use soluble class I MHC molecules to inhibit allogeneic T cell responses in vitro or in vivo. In general, soluble class I molecules have not effectively inhibited alloreactive T cell responses. Failure to observe inhibition ofT cell function with soluble MHC molecules may relate to the requirement for divalency to induce T cell anergy.

Divalency of the MHC molecules appears to be important for signal delivery to the T cell, including both activating and inhibitory signals. Further, T cell priming requires stimulation via the TCR and an additional second signal generally delivered by an antigen presenting cell. In the absence of a second signal, T cell hyporesponsiveness may result.

There remains a need for more effective immunotherapies for the modulation (e.g., enhancement or suppression) ofT cell mediated immunity. Therapies are needed to induce a sufficiently potent, antigen-specific, cell-mediated immune response which will either prevent a disease process such as an infection or tumor from becoming established, or will eliminate, or ameliorate a symptom, of an infection or tumor which has already become established in an individual. Therapies are also needed to prevent or suppress an autoimmune response or disease and to prevent or suppress transplant rejection in an individual.

All publications and patient applications cited herein are hereby incorporated by reference in their entirety.

### BRIEF SUMMARY OF THE INVENTION

The invention is directed to an antigen-scaffold complex comprising 15 or more different antigens, each of which comprise at least one MHC class I or MHC class II epitope. Accordingly, in one embodiment, the antigen-scaffold complex complexes more than 100 different antigens.

In some embodiments, the antigen-scaffold complex comprises the antigens coupled to a scaffold molecule, such as, for example, scaffold molecules of agarose, dextran or polylysine. In other embodiments, the antigen-scaffold complex comprises the antigens encapsulated in a liposome or coupled to the surface of a microccarrier particle.

In some embodiments, the antigens of the antigen-scaffold complex are peptides. In other embodiments, the antigen peptides of the complex include particular amino acid residues that effect intracellular processing, such as, for example, additional hydrophobic amino acid residues or additional basic amino acid residues at the carboxy termini, additional 1-3 lysine residues, an additional alanine-proline sequence and/or linker sequences containing proteolytic substrates.

In some embodiments, the antigen-scaffold complex further comprises a targeting ligand, such as for example, a targeting ligand that binds to a molecule selected from the group consisting oflangerin, DEC-205, DC-SIGN, TLR-3 and TLR-9. In some embodiments, the antigen-scaffold complex further comprises a D-type immunostimulatory oligonucleotide.

In another aspect, the invention is directed to a composition comprising a multiplicity of antigen∼scaffold complexes, in which the composition comprises 15 or more different antigens coupled to the complexes, where each antigen comprises at least one MHC class I or MHC class II epitope. Accordingly, in one embodiment, the composition comprising the antigen-scaffold complexes contains more than 100 different antigens.

In some embodiments, the invention is directed to compositions containing the antigen-scaffold complexes and further containing a pharmaceutically acceptable excipient and/or an adjuvant or innmmostimulatory agent.

In another aspect, the present invention is directed to methods of modulating an immune response through administration of the antigen-scaffold complexes and/or through administration of cells stimulated with the antigen-scaffold complexes of the invention.

Accordingly, in some embodiments, the invention includes methods of treating an infectious disease in an individual and methods of vaccinating an individual against an infectious agent through administering an antigen-scaffold complex in which the antigens are antigens of the infectious agent and wherein the complex is administered in an amount effective to stimulate a T cell response to the infectious agent.

In other embodiments, the invention includes methods of treating a cancer in an individual and methods of vaccinating an individual against a cancer through administering an antigen-scaffold complex in which the antigens are expressed on the cancer cells and wherein the complex is administered in an amount effective to stimulate a T cell response to the cancer cells.

In other embodiments, the invention includes methods of treating cancer in an individual comprising isolating and culturing a population of cells to stimulate proliferation and maturation of dendritic cells in the population, contacting the dendritic cells with an antigen-scaffold complex containing antigens expressed on the cancer, removing CD25+/CD4+ cells from the population of cells, and administering the population of cells without CD25+/CD4+ cells to the individual in an amount effective to stimulate a T cell response to the cancer cells.

In other embodiments, the invention includes methods of treating an autoimmune disorder in an individual comprising isolating and culturing a population of cells to stimulate proliferation and maturation of dendritic cells in the population, contacting the dendritic cells with an antigen-scaffold complex containing autoantigens, collecting CD25+/CD4+ cells from the population of cells, and administering the CD25+/CD4+ cells to the individual in an amount elective to suppress a symptom of the autoimmune disorder.

In another aspect, the invention is directed to methods of identifying immunodominant antigen epitopes in a population of antigens. In some embodiments, these methods comprising contacting a population of dendritic cells with antigen-scaffold complexes, collecting the population of cells, eluting antigens from the surface of the population of cells, and purifying the eluted antigens.

### DETAILED DESCRIPTION OF THE INVENTION

We have discovered that co-administration of many different antigens, including antigen peptides, is an effective way to modulate a desired immune response to the antigens and/or to cells expressing the antigens. Accordingly, the present invention offers complexes containing a minimum of 15 different MHC class I and/or MHC class II restricted antigens, including antigen peptides, that allow for co-administration of the antigens and result in a modulated (e.g., enhanced or suppressed) immune response as compared to administration of a single antigen or to the administration of the same antigens not in a complex. Antigens in the complexes of the invention differentially activate T helper (CD4+) cells, T cytotoxic (CD 8+) cells and T regulatory (CD4+/CD25+) cells. This differential activation is further developed upon activation and maturation of one or more professional antigen presenting cells (e.g., dendritic cells, Langerhans cels, interdigitating cells, and plasmacytoid cells).

The use of high capacity antigen complexes to induce a desired immune response offers distinct benefits for and advantages to administration of fewer antigens or to administration of the antigens not in a complex. The complexes allow co-delivery of many different MHC class I and/or class II restricted antigens to antigen presenting cells, such as dendritic cells. In some embodiments, a targeting ligand is coupled to the antigen complex to direct the complex to a particular subset of cells. For example, a targeting ligand which specifically interacts with a particular subset of dendritic cells is coupled, either directly or indirectly, to the complex so as to increase the likelihood of the antigen complex being directed to and taken up by the targeted cell.

The high number (15 or more) of antigens can be co-administered in complexes formulated with a variety of materials that provide a scaffold or an organization for the association of the antigens in a complex. For example, the antigen-scaffold complex can be made with multivalent scaffold molecules, liposomes, microspheres, ISCOMS, or high capacity carrier complexes. Accordingly, the complexes of the invention allow the concentration of antigen, particularly antigen peptides, so that more antigen can be administered to an individual,

Generally, the antigens of the antigen-scaffold complexes include a proteolytic substrate site or linker that facilitates delivery and processing of the antigens to an appropriate cellular component for presentation of the antigen in the context of an MHC molecule of the cell. For example, MHC class I restricted antigens are generally processed in a proteosome so an antigen which includes an MHC class I epitope may include a site or linker which is a substrate for a proteosome protease. MHC class II restricted antigens are generally processed in an endosome so an antigen which includes an MHC class II epitope may include a site or linker which is a substrate for an endosome protease.

The present invention is also directed to methods of modulating an immune response through administration of the antigen-scaffold complexes and/or through administration of cells stimulated with the antigen-scaffold complexes of the invention. In some embodiments, the methods also comprise administering to an individual (*in vivo*) or treating the cells (*in vitro*) with an adjuvant or immune stimulating agent before, during and/or after addition of the antigen-scaffold complexes. In some embodiments, methods of the invention induce desired immune responses involving both T helper and T cytotoxic cells and avoid an inflammatory response to the presenting dendritic cells and the antigen scaffold complexes.

Methods and compositions to enhance or suppress an immune response will depend on the particular immune response which the individual is in need of modulation. For example, particular methods of the invention are of use in enhancing an anti-cancer response and an anti-infectious agent response. Other methods of the invention are directed to suppression of an autoimmune response and/or disease or preventing transplant rejection.

Complexes and methods of the invention can also be of use in identification of immunodominant MHC epitopes. For example, after treating cells with the multi-antigen complexes of the invention, the antigen peptides associated with the MHC class I and class II molecules of the treated cells can be recovered and the peptides analyzed to identify the immunodominant MHC epitopes.

### Compositions of the invention

The antigen-scaffold complexes of the invention and/or compositions comprising antigen-scaffold complexes are designed to deliver many different antigens to a cell, tissue and/or individual. Accordingly, the complexes and/or composition comprising complexes contain more than 15 different antigens. In some embodiments, the complexes and/or compositions comprise more than 30 different antigens, more than 40 different antiges, more than 50 different antigens or more than 100 different antigens. In some embodiments, the complexes and/or compositions comprise 15 to about 100 different antigens, about 20 to about 100 different antigens, about 30 to about 90 different antigens, about 40 to about 80 different antigens, or about 40 to about 60 different antigens.

The antigen-scaffold complexes of the invention may be in a variety of forms. In some embodiments, antigens are associated with each other through their coupling to a common scaffold molecule. A "scaffold molecule" or "multivalent scaffold molecule" is a molecule containing multiple sites which allow for attachment of the antigens. In other embodiments, the antigen-scaffold complex contains antigens associated with each other through adsorption onto a common surface, such as a micro carrier particle. In other embodiments, the antigen-scaffold complex contains antigens associated with each other through association in or on liposomes or ISCOMs.

In embodiments in which the antigen-scaffold complex is made of antigens coupled to a multivalent scaffold molecule, the multivalent scaffold molecule generally possesses 15 or more sites for antigen coupling so that complexes with 15 or more antigens can be prepared. Examples of multivalent scaffold molecules include, but are not limited to, agarose, dextran, polylysine, polyarginine, ficoll, carboxymethylcellulose and polyvinyl alcohol, and derivatives thereof.

The principles of using multivalent scaffold molecules are well understood in the art. Generally, a scaffold molecule contains, or is derivatized to contain, appropriate binding sites for the antigens. In addition, or alternatively, antigens may be are derivatized to provide appropriate linkage groups. Examples of preferred linkage groups are described below.

Scaffold molecules may be biologically stabilized, *i.e.*, they exhibit an in vivo excretion half-life often of hours to days to months to confer therapeutic efficacy, and are preferably composed of a synthetic single chain of defined composition. They generally have a molecular weight in the range of about 200 to about 1,000,000, preferably any of the following ranges: from about 200 to about 500,000; from about 200 to about 200,000; from about 200 to about 50,000 (or less, such as 30,000).

In general, these scaffold molecules are made by standard chemical synthesis techniques. Some scaffold molecules must be derivatized and made multivalent, which is accomplished using standard techniques. Substances suitable for antigen-scaffold complex synthesis are available commercially.

Coupling of antigens to a multivalent scaffold molecule may be effected in any number of ways and may involve covalent and/or non-covalent interactions. Typically, coupling involves one or more crosslinking agents and/or functional groups on the antigens and scaffold molecule, Scaffolds and antigens must have appropriate (e.g., cooperative) linking groups. Linking groups are added to scaffolds using standard synthetic chemistry techniques. Linking groups may be added to polypeptide antigens using either standard solid phase synthetic techniques or recombinant techniques. Recombinant approaches may require post-translational modification in order to attach a linking group, and such methods are known in the art.

As an example of linking groups, polypeptides contain amino acid side chain moieties containing functional groups such as amino, carboxyl or sulfhydryl groups that serve as sites for coupling the polypeptide to the scaffold. Residues that have such functional groups may be added to the polypeptide if the polypeptide does not already contain these groups. Such residues may be incorporated by solid phase synthesis techniques or recombinant techniques, both of which are well known in the peptide synthesis arts. When the polypeptide antigen has a carbohydrate side chain(s) (or if the antigen is a carbohydrate), functional amino, sulfhydryl and/or aldehyde groups may be incorporated therein by conventional chemistry. For instance, primary amino groups may be incorporated by reaction of the oxidized sugar with ethylenediamine in the presence of sodium cyanoborohydride, sulfhydryls may be introduced by reaction of cysteamine dihydrochloride followed by reduction with a standard disulfide reducing agent, while aldehyde groups may be generated following periodate oxidation. In a similar fashion, the scaffold molecule may also be derivatized to contain functional groups if it does not already possess appropriate functional groups.

Typical methods for forming covalent coupling of the antigens to the antigen-scaffold complex, include formation of amides with the use of carbodiamides, or formation of sulfide linkages through the use of unsaturated components such as maleimide. Other coupling agents include, for example, glutaraldehyde, propanedial or butanedial, 2-iminothiolane hydrochloride, bifunctional N-hydroxysuccinimide esters such as disuccinimidyl suberate, disuccinimidyl tartrate, and the like. Linkage can also be accomplished by acylation, sulfonation, reductive amination, and the like. A multiplicity of ways to covalently couple a desired antigen to one or more components of the scaffold complex is well known in the art. Further, if the antigen is capable of direct adsorption to the scaffold complex, this too will effect its coupling.

The peptides useful in the present invention can be optionally flanked and/or modified at one or both of the N- and C-termini, as desired, by anmio acids from the naturally occurring sequences, amino acids added to facilitate linking to another peptide or to a lipid, other N- and C-terminal modifications, linked to carriers, etc., as further described herein. Additional amino acids can be added to the termini of a peptide to provide for modifying the physical or chemical properties of the peptide or the like. Amino acids such as tyrosine, cysteine, lysine, glutamic or aspartic acid, or the like, can be introduced at the C- or N-terminus of the peptide or oligopeptide. In addition, the peptide sequences can differ from the natural sequence by being modified by terminal-NH₂ acylation, e.g., by alkanoyl (C₁-C₂₀) or thioglycolyl acetylation, terminal-carboxy amidation, e.g., ammonia, methylamine, etc. In some instances these modifications may provide sites for linking to a support or other molecule.

In some instances, hydrophilic linkers of variable lengths are useful for connecting antigens to scaffold molecules. Suitable linkers include linear oligomers or polymers of ethylene glycol. Such linkers include linkers with the formula R¹S(CH₂CH₂O)ₙCHCH₂O(CH₂)ₘCO₂R² wherein n = 0-200, m = 1 or 2, R¹ = H or a protecting group such as trityl, R² = H or alkyl or aryl, *e.g.*, 4-nitrophenyl ester. These linkers are useful in connecting a molecule containing a thiol reactive group such as haloaceyl, maleiamide, etc., via a thioether to a second molecule which contains an amino group via an amide bond. These linkers are flexible with regard to the order of attachment, *i.e.*, the thioether can be formed first or last.

In some instances, avidin-biotm interactions are useful in coupling an antigen to a scaffold molecule. A biotin group can be attached, for example, to a moiety on the scaffold molecule and avidin or streptavidin incorporated into or attached onto the antigen. Alternatively, a biotin group can be attached to the antigen and avidin or streptavidin attached to the scaffold molecule. In either case, labeling one component with biotin and the other component with avidin or streptavidin allows for the formation of a non-covalently bound complex in which the antigen is coupled to a biotin-(strept)avidin linker which is coupled to a scaffold molecule. Methods and techniques for attaching biotin, avidin and streptavidin to molecules and cells are well known in the art. See, for example, O'Shannessey et al. (1984) Immunol, Lett. 8:273-277; O'Shannessy et al. (1985) J. Appl. Biochem. 7:347-355.

In embodiments in which the antigens are associated with the antigen-scaffold complex by adsorption onto a surface, the surface may be in the form of a carrier particle (for example, a microcarrier or nanoparticle) made with either an inorganic or organic core.

The term "microcarrier" refers to a particulate composition which is insoluble in water and which has a size of less than about 100 µm, preferably less than about 50-60 µm, preferably less than about 10 µm, preferably less than about 5 µm. Microcarriers include "nanocarriers", which are microcarriers have a size of less than about 1 µm, preferably less than about 500 nm. Microcarriers include solid phase particles such a particles formed from biocompatible naturally occurring polymers, synthetic polymers or synthetic copolymers, including agarose or cross-linked agarose. Solid phase microcarriers are formed from polymers or other materials which are non-erodible and/or non-degradable under mammalian physiological conditions, such as polystyrene, polypropylene, silica, ceramic, polyacrylamide, gold, latex, hydroxyapatite, dextran, and ferromagnetic and paramagnetic materials, Biodegradable solid phase microcarriers may be formed from polymers which are degradable (*e.g.*, poly(lactic acid), poly(glycolic acid) and copolymers thereof) or erodible (*e.g.*, poly(ortho esters such as 3,9-diethylidene-2,4,8,10-tetraoxaspiro[5.5]undecane (DETOS U) or poly(anhydrides), such as poly(anhydrides) of sebacic acid) under mammalian physiological conditions. Microcarriers may also be liquid phase (*e.g.*, oil or lipid based), such liposomes, iscoms (immume-stimulating complexes, which are stable complexes of cholesterol, phospholipid and adjuvant-active saponin) without antigen, or droplets or micelles found in oil-in-water or water-in-oil emulsions. Biodegradable liquid phase microcarriers typically incorporate a biodegradable oil, a number of which are known in the art, including squalene and vegetable oils. Microcarriers are typically spherical in shape, but microcarriers which deviate from speherical shape are also acceptable (*e.g.*, ellipsoidal, rod-shaped, etc.). Due to their insoluble nature, microcarriers are filterable from water and water-based (aqueous) solutions.

Examples of nanoparticles include, but are not limited to, nanocrystalline particles, nanoparticles made by the polymerization of alkylcyanoacrylates and nanoparticles made by the polymerization of methylidene malonate. Additional surfaces to which antigens may be adsorbed include, but are not limited to, activated carbon particles and protein-ceramic nanoplates.

Adsorption ofpolypeptides to a surface for the purpose of delivery of the adsorbed molecules to cells is well known in the art. See, for example, Douglas et al. (1987) Crit. Rev. Ther. Drug. Carriers Syst. 3.233-261; Hagiwara et al. (1987) In Vivo 1:241.252; Bousquet et al. (1999) Pharm. Res. 16:141-147. Preferably, the material comprising the adsorbent surface is biodegradable. Adsorption of antigens to a surface may occur through non-covalent interactions, including ionic and/or hydrophobic interactions.

In general, characteristics ofnanoparticles, such as surface charge, particle size and molecular weight, depend upon polymerization conditions, monomer concentration and the presence of stabilizers during the polymerization process (Douglas et al., 1987, *Supra*). For example, antigens of negative charge can adsorb directly to cationic surfaces of a microparticle. The surface of carrier particles may be modified, for example, with a surface coating, to allow or enhance adsorption of the antigens. Carrier particles with adsorbed antigens maybe further coated with other substances. The addition of such other substances may, for example, prolong the half-life of the particles once administered to the subject and/or may target the particles to a specific cell type or tissue, as described herein.

Nanocrystalline surfaces to which antigens may be adsorbed have been described. Another adsorbent surface are nanoparticies made by the polymerization of alkylcyanoacrylates. Alkylcyanoacrylates can be polymerized in acidified aqueous media by a process of anionic polymerization. Depending on the polymerization conditions, the small particles tend to have sizes in the range of 20 to 3000 nm, and it is possible to make nanoparticles specific surface characteristics and with specific surface charges (Douglas et al., 1987, *Supra*). Another adsorbent surface are nanoparticles made by the polymerization of methylidene malonate. For example, as described in Bousquet et al., 1999, *Supra*, polypeptides adsorbed to poly(methylidene malonate 2.1.2) nanoparticles appear to do so initially through electrostatic forces followed by stabilization through hydrophobic forces.

In some embodiments, antigens are associated in an antigen-scaffold complex through the use of an encapsulating agent that can maintain the association of the antigens until the complex is available to the target. In some instances, the antigen-scaffold complex comprising antigens and encapsulating agent is in the form of oil-in-water emulsions, microparticles and/or liposomes. In some instances, the oil-in-water emulsions, microparticles and/or liposomes encapsulating the antigens are in the form of particles from about 0.04 µm to about 100 µm in size, preferably any of the following ranges: from about 0.1 µm to about 20 µm; from about 0,15 µm to about 10 µm; from about 0.05 µm to about 1.00 µm; from about 0.05 µm to about 0.5 µm.

Colloidal dispersion systems, such as microspheres, beads, macromolecular complexes, nanocapsules and lipid-based system, such as oil-in-water emulsions, micelles, mixed micelles and liposomes can provide effective antigen-scaffold complexes.

The encapsulation composition may further comprises any of a wide variety of components. These include, but are not limited to, alum, lipids, phospholipids, lipid membrane structures (LMS), polyethylene glycol (PEG) and other polymers, such as polypeptides, glycopeptides, and polysaccharides.

Polypeptides suitable for encapsulation components include any known in the art and include, but are not limited to, fatty acid binding proteins. Modified polypeptides contain any of a variety of modifications, including, but not limited to glycosylation, phosphorylation, myristylation, sulfation and hydroxylation, As used herein, a suitable polypeptide is one that will protect an antigen-scaffold complex to preserve the immunomodulatory activity thereof. Examples of binding proteins include, but are not limited to, albumins.

Other suitable polymers can be any known in the art of pharmaceuticals and include, but are not limited to, naturally-occurring polymers such as dextrans, hydroxyethyl starch, and polysaccharides, and synthetic polymers. Examples of naturally occurring polymers include proteins, glycopeptides, polysaccharides, dextran and lipids. The additional polymer can be a synthetic polymer, Examples of synthetic polymers which are suitable for use in the present invention include, but are not limited to, polyalkyl glycols (PAG) such as PEG, polyoxyethylated polyols (POP), such as polyoxyethylated glycerol (POG), polytrimethylene glycol (PTG) polypropylene glycol (PPG), polyhydroxyethyl methacrylate, polyvinyl alcohol (PVA), polyacrylic acid, polyethyloxazoline, polyacrylamide, polyvinylpyrrolidone (PVP), polyamino acids, polyurethane and polyphosphazene. The synthetic polymers can also be linear or branched, substituted or unsubstituted, homopolymeric, co-polymers, or block copolymers of two or more different synthetic monomers. The PEGs for use in encapsulation compositions of the present invention are either purchased from chemical suppliers or synthesized using techniques known to those of skill in the art.

The term "LMS", as used herein, means lamellar lipid particles wherein polar head groups of a polar lipid are arranged to face an aqueous phase of an interface to form membrane structures. Examples of the LMSs include liposomes, micelles, cochleates (i.e., generally cylindrical liposomes), microemulsions, unilamellar vesicles, multilamellar vesicles, and the like.

As used herein, a "liposome" or "lipid vesicle" is a small vesicle bounded by at least one and possibly more than one bilayer lipid membrane. Liposomes are made artificially from phospholipids, glycolipids, lipids, steroids such as cholesterol, related molecules, or a combination thereof by any technique known in the art, including but not limited to sonication, extrusion, or removal of detergent from lipid-detergent completes. A liposome can also optionally comprise additional components, such as a tissue targeting component. It is understood that a "lipid membrane" or "lipid bilayer" need not consist exclusively of lipids, but can additionally contain any suitable other components, including, but not limited to, cholesterol and other steroids, lipid-soluble chemicals, proteins of any length, and other amphipathic molecules, providing the general structure of the membrane is a sheet of two hydrophilic surfaces sandwiching a hydrophobic core.

Processes for preparing liposomes containing antigens are known in the art. The lipid vesicles can be prepared by any suitable technique known in the art. Methods include, but are not limited to, microencapsulation, microfluidization, LLC method, ethanol injection, freon injection, the "bubble" method, detergent dialysis, hydration, sonication, and reverse-phase evaporation. Reviewed in Watwe et al. (1995) Curr. Sci. 68:715-724. Techniques maybe combined in order to provide vesicles with the most desirable attributes. Antigens may be included in the liposomal membrane if the properties of the antigens are suitable. For example, if an antigen contains a highly lipophilic portion, it may itself be embedded in the surface of the liposome.

Antigens of the complexes of the invention may be modified in order to facilitate antigen processing and longevity, and to increase antigen presentation on the cell surface.

For example, antigens and antigen peptides used in the antigen-scaffold complexes of the invention will preferably include particular sequences or linker portions that are proteolytic substrates. The proteolytic substrate sequences or linkers are used to facilitate delivery of the antigens to the appropriate cellular compartment(s) and processing of the antigens for presentation in the context of an MHC molecule of the cell. MHC class I restricted antigens are generally processed in a proteosome and then associated with an MHC class I molecule and with β2 microglobulin. This trimolecular complex is then transported to the cell surface, where antigen presentation occurs. Accordingly, peptide sequences or linkers that are substrates for proteosome proteases, such as chymotrypsin, trypsin and caspase, are present in or added to antigen peptides and/or antigens that include MHC class I restricted epitopes. MHC class II restricted antigens are generally processed in an endosome and then associated with an MHC class II molecule. The MHC class II-antigen complex is then transported to the cell surface, where antigen presentation occurs. Accordingly, peptide sequences or linkers that are substrates for endosome proteases (endopeptidates) are present in or added to antigen peptides and/or antigens that include MHC class II restricted epitopes. Examples of endosomal proteases include cathepsin D, cathepsin S and cathepsin L. Cathepsin S is generally the predominant endosomal protease in dendritic cells.

Peptide sequences that are proteolytic substrates for proteosomal and endosomal proteases are known in the art. For example, sequences for use as a proteolytic substrate linker for proteosomal proteases include, but are not limited to, Suc-Ala-Glu ∼ peptide, Leu-Leu-Leu ∼ peptide, and Leu-Leu-Glu - peptide. See, for example, Aki et al. (1994) J. Biochem. 115:257-269; Tsubuki et al. (1993) Biochem. Biophys. Res. Commun 196:1195-1201. Accordingly, such sequences are included in proteolytic linkers for the antigens with class I epitopes or are found in the antigens with class I epitopes. Examples of sequences for use as a proteolytic substrate linker for endosomal proteases include, but are not limited to, Arg-Gly-Phe ∼ Phe-peptide and Arg-Gly-Phe ∼ Phe-Ala-Pro-peptide (substrates for cathepsin D), Val∼Val-Arg∼ peptide and Val-Val-Arg ∼ Ala-Pro-peptidc (substrates for cathepsin S) and Leu-Phe-Arg ∼ peptide and Leu-Phe-Arg ∼ Ala-Pro-peptide (substrates for cathepsin L). See, for example, Scarborough et al. (1993) Protein Sci. 2:264-276; Claus et al. (1998) J. Biol, Chem. 273:9842-9851. Accordingly, such sequences are included in proteolytic linkers for the antigens with class II epitopes or are found in the antigens with class II epitopes. In the sequences listed above the "∼" symbol indicates the protease cleavage site. In some instances a Gly or Ala may be added following the cleavage site.

In the antigen-scaffold complex, antigens may be part of the complex through linkage to other antigens. For example, concatenated antigen peptides, particularly with cleavable linkers between them, could be coupled to a scaffold molecule or included in another way with an antigen-scaffold complex. Linking antigens to antigens would allow for higher concentration of antigen per amount of complex.

Whereas specific proteolytic cleavage at particular sites as described above is desirable for antigens of the invention, general proteolytic degradation of the antigen or antigen peptide is typically not desirable. Accordingly, antigens and antigen peptides may be modified to inhibit general proteolytic degradation. For example, antigens of the complexes may include, or be modified to include, the sequence Ala-Pro before the MHC class I or class II epitope. The Ala-Pro sequence helps prevent degradation of the peptide and thus, increases epitope longevity and time for presentation on the cell surface. When used in the antigen in conjunction with a proteolytic linker, the Ala-Pro sequence would be located between the MHC epitope and the proteolytic linker. Examples of such antigen peptides with proteolytic linkers and/or Ala-Pro sequences are found below.

In some embodiments, with MHC class I restricted antigens in particular, the antigen may be modified or selected to include sites for ubiquitination. Multiubiquinated antigens are directed to the proteosome for cleavage into peptides and for encounter with MHC class I molecules. Hershko et al. (1998) Anm. Rev. Biochem. 67:425-530. Ubiquitination of a polypeptide occurs when a conjugating enzyme catalyzes formation of a peptide bond between ubiquity and the side chain -NH₂ of a lysine residue in the target polypeptide. Additional ubiqnitin molecules may then be added to form a multiubiquitinylated polypeptide. Accordingly, antigens may be modified by the addition of one to three lysine residues and/or selected to include one to three lysine residues so that ubiquitination can occur and the antigen directed to a proteosome.

An MHC class I antigen may also be modified or selected to include additional hydrophobic or basic amino acid residues at its carboxy terminus. Such residues help favor processing by the transporter associated with antigen processing (TAP) pathway, as described, for example, in Goldberg et al. (2002) Mol. Immunol. 39:147-164.

### Antigens

As used herein, the term "antigen" means a substance that is recognized and bound specifically by an antibody or by a T cell antigen receptor. Antigens can include peptides, proteins, glycoproteins, polysaccharides, complex carbohydrates, sugars, gangliosides, lipids and phospholipids; portions thereof and combinations thereof The antigens can be those found in nature or can be synthetic. Preferably, antigens suitable for administration in the complexes include any MHC class I or MHC class II epitopes. Haptens are included within the scope of "antigen." A hapten is a low molecular weight compound that is not immunogenic by itself but is rendered immunogenic when conjugated with an immunogenic molecule containing antigenic determinants. Small molecules may need to be haptenized in order to be rendered antigenic. Preferably, antigens of the present invention include peptides, lipids (e.g. sterols, fatty acids, and phospholipids), polysaccharides, gangliosides and glycoproteins.

As used herein, the term "peptide" are polypeptides that are of sufficient length and composition to effect a biological response, *e.g.*, antibody production or cytokine activity whether or not the peptide is a hapten, or presentation in the context of an MHC molecule. Typically, the peptides are at least six amino acid residues in length. The term "peptide" further includes modified amino acids (whether or not naturally or non-naturally occurring), such modifications including, but not limited to, phosphorylation, glycosylation, pegylation, lipidization and methylation.

"Antigenic peptides" or "antigen peptides" can include purified native peptides, synthetic peptides, recombinant proteins, crude protein extracts, attenuated or inactivated viruses, cells, micro-organisms, or fragments of such peptides. An "antigenic peptide" or "antigen polypeptide" accordingly means all or a portion of a polypeptide which exhibits one or more antigenic properties (e.g., binds specifically to an antibody or a T cell receptor).

Many MHC class I and class II antigenic peptides and polypeptides are known and available in the art (see, for example, U.S. Pat. No. 6,419,931); others can be identified using conventional techniques as known in the art and described herein. For immunization against tumor formation or treatment of existing tumors, antigens can include tumor cells (live or irradiated), tumor cell extracts, or protein subunits or peptides of tumor antigens such as Her-2/neu, Mart1, carcinoembryonic antigen (CEA), gangliosides, human milk fat. globule (HMFG), mucin (MUC1), MAGE antigens, BAGE antigens, GAGE antigens, gp100, prostate specific antigen (PSA), and tyrosinase.

Exemplary tumor antigen peptides, and the MHC molecules with which they are presented, are listed in Tables 1 and 2. Additional tumor antigens for use in the present invention are known in the art and described, for example, in Renkvist et al. (2001) Cancer Immunol. Immunother. 50:3-15; Robbins et al. (1996) Curr. Opin. Immunol. 8:628-636; Scanlan et al. (2002) Immunol. Rev. 188:22-32; Wang (1999) J. Mol. Med. 77:640-655.

**TABLE 1: MHC Class II Antigen Peptides**

| Antigen Peptide | Antigen | MHC class II allele | Reference |
|---|---|---|---|
| WNRQLYPEWTEAQRLD | gp100 | HLA-DR-B1*0401 | Kierstead et al. (2001) Brit. J. Cancer 85:1738-1745 |
| IYRRRLMKQDFSVPQLPHS | gp100 | HLA-DR-B1*0401 | |
| QNILLSNAPLGPQFP | tyrosinase | HLA-DR-B1*0401 | |
| YGQMKNGSTPMFNDlNIYDL | tyrosinase | HLA-DR-B1*0401 | |
| ALHIYMDGTMSQVQGSA | tyrosinase | HLA-DR-B1*0401 | |
| RNGYRALMDKSLHVGTQCALTRR | MART-1 | HLA-DR-B1*0401 | Zarour et al. (2000) Proc. Natl. Acad. Sci. USA 97:400-405 |
| POVLLKHFTVSGNILTIRLTAADHR | NY-ESO-1/ LAGE-2 | HLA-DR-B1*0401 | Zarour et al. (2000) Cancer Res. 60:4946-4952 |
| PGVLLKEFTVSG | NY-ESO-1 | HLA-DR-B1*0401 | Zeng et al. (2000) J. Immunol. 165:1153-1159 |
| ESEFQAALSRKVALK | MAGE-6 | HLA-DR-B1*0401 | Tatsumi et al. (2003) Clin. Cancer Res. 9:947-954 |
| LLKYRAREPVTKAEMLGSVVGNWQ | MAGE-6 | HLA-DR-B1*0401 | |
| IFSKASDSLQLVFGIE | MAGE-6 | HLA-DR-B1*0401 | |
| LTQYFVQENYLLEYRQVPG | MAGE-6 | HLA-DR-B1*0401 | |
| QNILLSNAPLGPQFP | tyrosinase | HLA-DR4 | Topalian et al. (1996) J. Exp. Med. 183:1965-1971 |
| SYLQDSDPDSFQD | tyrosinase | HLA-DR4 | |
| WNRQLYPEWTEAQRLD | gp100 | HLA-DR4 | Li et al.(1998) Cancer Immunol. Immunother. 47:32-38 |
| PGVLLKEFTVSGNILTIRLT | LAGE-2 | HLA-DR4 | Jager et al. (2000) J. Exp. Med. 191:625 |
| AADHRQLQLSISSCLQQL | LAGE-2 | HLA-DR4 | |
| VIFSKASSSLQL | MAGE-A3 | HLA-DR4 | Kobayashi et al. (2001) Cancer Res. 61:4773 |

**TABLE 2: MHC Class I Antigen Peptides**

| Antigen Peptide | Antigen | MHC class I allele | Reference |
|---|---|---|---|
| RVAALARDA | 707-AP | HLA-A2 | Morioka et al. (1995) Mol. Immunol. 32:573-581 |
| AAGIGILTV | MART-1/ Melan-A | HLA-A2 | Coulie et al. (1994) J. Exp. Med. 180:35-42 |
| EAAGIGILTV | MART-1/ Melan-A | HLA-A2 | Schneider et al. (1998) Int. J. Cancer 75:451-458 |
| ILTVILGVL | MART-1/ Melan-A | HLA-A2 | Castelli et al. (1995) J. Exp. Med. 181:363-368 |
| AMLGTHTMEV | gp100 | HLA-A2 | Tsai et al. (1997) J. Immunol. 158:1796-1802 |
| MLGTHTMEV | gp100 | HLA-A2 | |
| SLADTNSLAV | gp100 | HLA-A2 | |
| ITDQVPFSV | gp100 | HLA-A2 | Kawakami (1995) J. Immunol. 154:3961 |
| LLDGTATLRL | gp100 | HLA-A2 | Kawakami (1994) Proc Natl. Acad Sci USA 91:3515 |
| KMVELVHFL | MAGE-A2 | HLA-A2 | Visseren et al. (1997) Int. J. Cancer 73:125-1.30 |
| YLQLVFGIEV | MAGE-A2 | HLA-A2 | |
| FLWGPRALV | MAGE-A3 | HLA-A2 | Van der Bruggen et al. (1994) Eur. J. Immunol. 24:3038-3043 |
| GVYDGREHTV | MAGE-A4 | HLA-A2 | Duffour et al. (1999) J. Immunol. 29:3329 |
| FLWGPRAYA | DAM-6, -10 | HLA-A2 | Fleischhauer et al. (1998) Cancer Res. 58:2969 |
| SLLMWTTQCFL | NY-ESO-1 | HLA-A2 | Jager et al. (1998) J. Exp. Med. 187:265 |
| YMDGTMSQV | tyrosinase | HLA-A2 | Wolfel et al. (1994) Eur. J. Immunol. 24:759 |
| MLLAVLYCL | tyrosinase | HLA-A2 | |
| YMNGTMSQV | tyrosinase | HLA-A2 | Visseren et al. (1995) J. Immunol. 154:3991 |
| SVYDFFVWL | TRP-2 | HLA-A2 | Parkhurst et al. (1998) Cancer Res. 58:4895 |

In some embodiments, the antigen is from an infectious agent, including protozoan, bacterial, fungal (including unicellular and multicellular), and viral infectious agents. Examples of suitable viral antigens are described herein and are known in the art. Bacteria include *Hemophilus influenza, Mycobacterium tuberculosis* and *Bordetella pertussis*. Protozoan infectious agents include malarial plasmodia, *Leishmania* species, *Trypanosoma* species and *Schistosoma* species. Fungi include *Candida albicans*.

In some embodiments, the antigen is a viral antigen including viral a protein and/or peptide. Viral polypeptide antigens include, but are not limited to, HIV proteins such as HIV gag proteins (including, but not limited to, membrane anchoring protein, core capsid protein and nucleocapsid protein), HIV polymerase, hepatitis B polymerase, hepatitis B core protein, hepatitis B envelope protein, hepatitis C core antigen, hepatitis C NS1, NS3, NS4 and NS5 antigen, hepatitis C envelope antigen, human papillomavirus (HPV) E6 and E7 antigens (including, but not limited to, HPV16-E6 and HPV16-E7 polypeptides), and the like. Other examples of antigen polypeptides are group- or sub-group specific antigens, which are known for a number of infectious agents, including, but not limited to, herpes simplex viruses and poxviruses. See also, for example, U.S. Pat. No. 6,419,931.

Attenuated and inactivated viruses are suitable for use herein as the antigen. Preparation of these viruses is well-known in the art and many are commercially available, for example, polio virus, hepatitis A virus, measles virus, mumps virus and rubella virus (see, e.g., Physicians' Desk Reference (1998) 52nd edition, Medical Economics Company, Inc.). Additionally, attenuated and inactivated viruses such as HIV-1, HIV-2, herpes simplex virus, hepatitis B virus, rotavirus, human and non-human papillomavirus and slow brain viruses can provide peptide antigens.

Autoimmune associated disorders for which the antigens of the invention may be employed to relieve the symptoms of, treat or prevent the occurrence or reoccurrence of include, for example, multiple sclerosis (MS), rheumatoid arthritis (RA), Sjogren syndrome, scleroderma, polymyositis, dermatomyositis, systemic lupus erythematosus, juvenile rheumatoid arthritis, ankylosing spondylitis, myasthenia gravis (MG), bullous pemphigoid (antibodies to basement membrane at dermal-epidermal junction), pemphigus (antibodies to mucopolysaccharide protein complex or intracellular cement substance), glomerulonephritis (antibodies to glomerular basement membrane), Goodpasture's syndrome, autoimmune hemolytic anemia (antibodies to erythrocytes), Hashimoto's disease (antibodies to thyroid), pernicious anemia (antibodies to intrinsic factor), idiopathic thrombocytopenic purpura (antibodies to platelets), Grave's disease, and Addison's disease (antibodies to thyrogbulin), and the like.

The autoantigens associated with a number of these diseases have been identified. For example, in experimentally induced autoimmune diseases, antigens involved in pathogenesis have been characterized: in arthritis in rat and mouse, native type-II collagen is identified in collagen-induced arthritis, and mycobacterial heat shock protein in adjuvant arthritis; thyroglobulin has been identified in experimental allergic thyroiditis (EAT) in mouse; acetyl choline receptor (AChR) in experimental allergic myasthenia gravis (EAMG); and myelin basic protein (MBP) and proteolipid protein (PLP) in experimental allergic encephalomyelitis (EAE) in mouse and rat. In addition, autoantigens have been identified in humans: type-II collagen in human rheumatoid arthritis; and acetyl choline receptor in myasthenia gravis.

Preferably the antigens are peptides. In some embodiments, however, an antigen may be a lipid (e.g., sterol excluding cholesterol, fatty acid, and phospholipid), polysaccharide such as those used in H. influenza vaccines, ganglioside and glycoprotein. These can be obtained through several methods known in the art, including isolation and synthesis using chemical and enzymatic methods. In certain cases, such as for many sterols, fatty acids and phospholipids, the antigenic portions of the molecules are commercially available.

Antigenic peptides can be native or synthesized chemically or enzymatically. Any method of chemical synthesis known in the art is suitable. Solution phase peptide synthesis can be used to construct peptides of moderate size or, for the chemical construction of peptides, solid phase synthesis can be employed. Atherton et al. (1981) Hoppe Seylers Z. Physiol. Chem. 362:833∼839. Proteolytic enzymes can also be utilized to couple amino acids to produce peptides. Kullmann (1987) Enzymatic Peptide Synthesis, CRC Press, Inc. Alternatively, the peptide can be obtained by using the biochemical machinery of a cell, or by isolation from a biological source. Recombinant DNA techniques can be employed for the production of peptides. Hames et al. (1987) Transcription and Translation: A Practical Approach, IRL Press. Peptides can also be isolated using standard techniques such as affinity chromatography, MHC class I and II epitopes can also be modified to increase their biological effect. For example, the peptides can contain D-amino acids to increase their resistance to proteases and thus extend their serum half-life.

Although in some cases, the peptide will preferably be substantially free of other naturally occurring viral, bacterial parasitic, tumor or self proteins and fragments thereof, in some embodiments the peptides can be synthetically conjugated to native fragments or particles. The term peptide is used interchangeably with polypeptide in the present specification to designate a series of amino acids connected one to the other by peptide bonds between the alpha-amino and alpha-carboxy groups of adjacent amino acids. The polypeptides or peptides can be a variety of lengths, either in their neutral (uncharged) forms or in forms which are salts, and either free of modifications such as glycosylation, side chain oxidation, or phosphorylation or containing these modifications, subject to the condition that the modification not destroy the biological activity of the polypeptides as herein described.

The terms "homologous", "substantially homologous", and "substantial homology" as used herein denote a sequence of amino acids having at least 50% identity wherein one sequence is compared to a reference sequence of amino acids. The percentage of sequence identity or homology is calculated by comparing one to another when aligned to corresponding portions of the reference sequence.

In some instances, the antigenic peptide will be as small as possible while still maintaining substantially all of the biological activity of a larger peptide. In some instances, it may be desirable to optimize peptides of the invention to a length of eight to twelve amino acid residues, more usually nine or ten amino acid residues, commensurate in size with endogenously processed antigen peptide that is bound to MHC class I molecules on the cell surface. An MHC class II peptide will typically comprise from about six to about thirty amino acids and will contain a T helper cell inducing epitope. See generally, Schumacher et al. (1991) Nature 350:703-706; Van Bleek et al. (1990) Nature 348:213-216; Rotzschke et al. (1990) Nature 348:252-254; and Falk et al. (1991) Nature 351:290-296. By biological activity of a CTL inducing peptide is meant the ability to bind an appropriate MHC molecule and, in the case of peptides useful for stimulating CTL responses, induce a CTL response against the selected antigen or antigen mimetic. By a CTL response is meant a CD8+ T lymphocyte response specific for an antigen of interest, wherein CD8+, MHC class I-restricted T lymphocytes are activated. By a T helper cell response is meant a CD4+ T lymphocyte response wherein CD4+ T lymphocytes are activated. The T helper cells stimulated by the T helper cell-inducing peptide can be the T helper 1 (Th1) and/or T helper 2 (Th2) phenotype, for example. The activated T helper lymphocytes will secrete a variety of products, including, for example, interleukin-2, which may facilitate expression of the T cell receptor and promote recognition by activated CTLs.

Examples of synthesized antigen peptides containing various portions or amino acid residues described herein include those listed in below in the paragraph. In addition to an HLA-DR4 restricted epitope, these peptides include a proteolytic substrate linker, an additional Gly or Ala and/or an additional Ala-Pro sequence, as described herein. These peptides are also listed with a resin used for solid phase synthesis methodology, i.e., a Rink resin or a Wang resin, as known in the art. The following antigen peptides are from the antigen tyrosinase and are presented by the MHC allele HLA-DR4:
G-VVR∼QNILLSNAPLGPQFP-Rink Resin; G-VVR∼QNILLSNAPLGPQFP(F[Br])-Rink Resin; G-VVR∼QNILLSNAPLGPQFP-Wang Resin; G-VVR∼QNILLSNAPLGPQFP(F[Br])-Wang Resin; G-VVR∼(AP)QNILLSNAPLGPQFP-Rink Resin;
G-VVR∼(AP)QNILLSNAPLGPQFP(F[Br])-Rink Resin;
G-VVR∼(AP)QNILLSNAPLGPQFP-Wang Resin;
G-VVR∼(AP)QNILLSNAPLGPQFP(F[Br])-Wang Resin;
G-VVR∼SYLQDSDPDSFQD-Rink Resin; G-VVR∼SYLQDSDPDSFQD(F[Br]-Rink Resin;
G-VVR∼SYLQDSDPDSFQD-Wang Resin; G-VVR∼SYLQDSDPDSFQD(F[Br])-Wang Resin;
G-VVR∼(AP)SYLQDSDPDSFQD-Rink Resin; G-VVR∼(AP)SYLQDSDPDSFQD(F[Br])-Rink Resin; G-VVR∼(AP)SYLQDSDPDSFQD-Wang Resin; and
G-VVR∼(AP)SYLQDSDPDSFQD(F[Br])-Wang Resin. The following antigen peptides are from the antigen MAGE-A3 and are presented by the MHC allele HLA-DR4:
G-VVR∼VIFSKASSSLQL-Rink Resin;G-VVR∼VIFSKASSSLQL(F[Br])-Rink Resin;
G-VVR~VIFSKASSSLQL-Wang Resin; G-VVR∼VIFSKASSSLQL(F[Br])-Wang Resin;
G-VVR~(AP)VIFSKASSSLQL-Rink Resin; O-VVR∼(AP)VIFSKASSSLQL(F[Br])-Rink Resin;
G-VVR∼(AP)VIFSKASSSLQL-Wang Resin; and G-VVR∼(AP)VIFSKASSSLQL(F[Br])-Wang Resin. In the sequences listed above, the "∼" symbol indicates the protease cleavage site.

Generally, peptides are used in the methods and compositions of the present invention irrespective of the method or methods used to identify the MHC epitope. The MHC class I and/or class II epitope(s) contained in peptides can be identified in one of several known ways. In those cases where antigen-specific T cell lines or clones are available, for example tumor-infiltrating lymphocytes (TIL) or virus-specific CTL, these cells can be used to screen for the presence of the relevant epitopes using target cells prepared with specific antigens. Such. targets can be prepared using random, or selected synthetic peptide libraries, which would be utilized, for example, to sensitize the target cells for lysis by the CTL. Another approach to identify the relevant MHC epitope when T cells are available is to use recombinant DNA methodologies. Gene, or preferably cDNA, libraries from T cell responsive targets or T cell-susceptible targets are first prepared and transfected into non-responsive or non-susceptible target cells. This allows the identification and cloning of the gene coding the protein precursor to the peptide containing the MHC epitope. The second step in this process is to prepare truncated genes from the relevant cloned gene, in order to narrow down the region that encodes for the MHC epitope. This step is optional if the gene is not too large. The third step is to prepare synthetic peptides of approximately 10-20 amino acids of length, overlapping by 5 amino acid residues, which are used to sensitize targets for the T cells. When a peptide, or peptides, are shown to contain the relevant MHC epitope, smaller peptides can be prepared to establish the peptide of minimal size that contains the MHC epitope. These MHC class I epitopes are often contained within 9-10 residues.

Another way of identifying a peptide containing an MHC epitope, when T cells are present, is to elute the peptide with an acid or base. The peptides associated with MHC molecules are present on, for example, antigen presenting cells and cells lysed by CTLs. The eluted peptides are separated using a purification method such as HPLC, and individual fractions are tested for their capacity to sensitize targets for CTL lysis or to activate T helper cells. When a fraction has been identified as containing the MHC peptide, it is further purified and submitted to sequence analysis. The peptide sequence can also be determined using tandem mass spectrometry. A synthetic peptide may then be prepared and tested with the T cell to corroborate that the correct sequence and peptide have been identified.

In some circumstances, where T cells are not available there are other means to identify potential MHC epitopes. These methods rely in the identification of MHC-binding peptides from known protein sequences. See, for example, U.S. Pat. Nos. 5,662,907 and 6,037,135. Briefly, the protein sequences for example from viral or tumor cell components are examined for the presence of MHC-binding motifs. These binding motifs which exist for each MHC allele, are conserved amino acid residues, usually at positions 2 (or 3) and 9 (or 10) in peptides of 9-10 amino acid residues long. Synthetic peptides are then prepared of those sequences bearing the MHC binding motifs, and subsequently are tested for their ability to bind to MHC molecules. The MHC binding assay can be done either using cells which express high number of empty MHC molecules (cellular binding assay), or using purified MHC molecules. Lastly, the MHC binding peptides are then tested for their capacity to induce a CTL response or a T helper cells response in naive individuals, either in vitro using human lymphocytes, or in vivo using HLA-transgenic animals. These CTL are tested using peptide-sensitized target cells, and targets that naturally process the antigen, such as viral infected cells or tumor cells. For example, a HLA-A1-restricted. CTL epitope for the tumor-associated antigen MAGE-3 has been identified using this approach (U.S. Pat. No. 5,662,907). Another approach to identify the relevant MHC class I epitopes is by combining predictions of MHC class I binding affinities with predictions of TAP pathway transport efficiency as described, for example, by Peters et al. (2003) J. Immunol. 171:1741-1749.

### Targeting ligands

In some embodiments, the invention is directed to antigen-scaffold complexes containing tissue or cell targeting ligands. Such targeting ligand are components of an antigen-scaffold complex that enhance accumulation of the complex at certain tissue or cellular sites in preference to other tissue or cellular sites when administered to an intact animal, organ, or cell culture. Inclusion of the targeting ligands with the complexes result in the localization and binding of the complexes to molecular epitopes, i.e., receptors, lipids, peptides, cell adhesion molecules, polysaccharides, biopolymers and the like, presented on the surface membranes of targeted cells or tissues or within extracellular or intracellular matrix. Generally, the ligand specifically binds to a cellular epitope or receptor. A wide variety of targeting ligands can be used including, but not limited to, an antibody, a fragment of an antibody, a polypeptide such as small oligopeptide, a large polypeptide or a protein having three dimensional structure, a peptidomimetic, a polysaccharide, an aptamer, a lipid, a carbohydrate, a nucleic acid, a small molecule such as a drug, hormone or hapten, a lectin or a combination thereof. Antibodies with specificity toward cell type-specific cell surface markers are known in the art and are readily prepared by methods known in the art. Since a targeting component is generally accessible from outside the liposome, and is therefore preferably either bound to the outer surface or inserted into the outer lipid bilayer when the complex is made with a liposome.

The antigen-scaffold complexes can be targeted to any cell type toward which the complex is to be directed, e.g., a cell type which can present antigen and/or participate in an immune response. Such target cells and organs include, but are not limited to, antigen presenting cells (APCs), such as dendritic cells, lymphocytes and macrophages, lymphatic structures, such as lymph nodes and the spleen, and nonlymphatic structures, particularly those in which dendritic cells are found. In some embodiments, the antigen-scaffold complexes are preferably targeted to and taken up by dendritic cells or subpopulations of dendritic cells, e.g., Langerhans cells, plasmacytoid cells, interdigitating cells, and/or interstitial cells. In some embodiments, the antigen-scaffold complexes are preferentially not directed to macrophage.

The term "ligand" as used herein is intended to refer to a targeting molecule that binds specifically to another molecule of a biological target separate and distinct from the antigen-scaffold complex itself. The reaction does not require nor exclude a molecule that donates or accepts a pair of electrons to form a coordinate covalent bond with a metal atom of a coordination complex. Thus a ligand may be attached covalently for direct-conjugation or non covalently for indirect conjugation to the antigen-scaffold complex.

Accordingly, targeting ligands that interact with molecular epitopes on target tissues or cells include, but are not limited to, natural or non-natural ligands of the molecular epitope (e.g., a receptor) and antibodies that bind the molecular epitope. Targeting ligands of the present invention include, but are not limited to, those that interact with langerin (CD207), multilectin receptor (such as DEC-205 in mice), DC-SIGN (dendritic cell-specific ICAM-3 grabbing non-integrin), Fc receptor and Toll-like receptors (TLR). Accordingly, targeting ligands that interact with these molecules include, but are not limited to, anti-langenn antibodies (such as DCGM4 (Valladeau et al. (1999) Eur. J. Immunol. 29-2695-2704)), mannose, mannan, anti-multilectin receptor antibodies, anti-DEC-205 antibodies, anti-DC-SIGN antibodies, anti-Fc receptor antibodies, FcγRII (CD32), FcαR (CD89), FcγRI, FcεRI/IL-3Rα (CD123), TLR-3 ligand and TLR-9 ligands, such as CpG-containing oligonucleotides. The use of mannose and of a targeting ligand that interacts with DEC-205 or DC-SIGN preferentially targets the complex to various dendritic cells (Bonifaz et al. (2002) J. Exp. Med. 196: 1627-163 8; Engering et al. (2002) J. Immunol. 168:2118-2126). The use of a targeting ligand that interacts with langerin preferentially targets the complex to Langerhans cells (Takahara et al. (2002) Int. Immunol. 14:433-444). The use of FcγRII (CD32) or FcαR (CD89) as a targeting ligand that interacts with Fc receptor preferentially targets the complex to monocyte-derived cells. The use ofFcyRI or FcεRI/IL-3Rα (CD123) preferentially targets the complex to Langerhans cells (Guermonprez et al. (2002) Ann. Rev, Immunol. 20:621-667). The use of a ligand that interacts with TLR-3, such as TLR-3 ligand and polyinosine-polycytidylic acid (polyI:C), preferentially targets the complex to various dendritic cells (Alexopoulou et al. (2001) Nature 413:732-738). The use of a ligand that interacts with TLR-9, such as oligonucleotides containing unmethylated CpG motifs, in particular "D"-type oligonucleotides, targets the complex to various dendritic cells, monocytes and other immune system cells (Klinman et al. (2002) Microbes and Infection 4:897-901). CpG containing D-type oligonucleotides include, for example, 5'-GGTGCATCGATGCAGGGGGG-3' (D 19) and 5'-GGTGCACCGGTGCAGGGGGG-3' (D29). D-type oligonucleotides associated with the complexes not only target the complexes but also facilitate endocytosis of the complexes and stimulate monocytes to mature into CD83+/CD86+ dendritic cells. Klinman et al., *Supra.*

Antibodies, particularly monoclonal antibodies, may be used as targeting ligands directed to any of a spectrum of desired molecular epitopes. Immunoglobin-γ (IgG) class monoclonal antibodies have been coupled to liposomes, emulsions and other particles to provide active, site-specific targeting. Generally, these proteins are symmetric glycoproteins (MW ca. 150,000 Daltons) composed of identical pairs of heavy and light chains. Hypervariable regions at the end of each of two arms provide identical antigen-binding domains. A variably sized branched carbohydrate domain is attached to complement-activating regions, and the hinge area contains particularly accessible interchain disulfide bonds that may be reduced to produce smaller fragments.

Preferably, monoclonal antibodies are used in the antibody compositions of the invention. Monoclonal antibodies specific for selected antigens on the surface of cells may be readily generated using conventional techniques (see, for example, U.S. Pat. Nos, RE 32,011, 4,902,614, 4,543,439, and 4,411,993). Hybridoma cells can be screened immunochemically for production of antibodies specifically reactive with an antigen, and monoclonal antibodies can be isolated. Other techniques may also be utilized to construct monoclonal antibodies (see, for example, Huse et al. (1989) Science 246:1275-1281; Sastry et al. (1989) Proc. Natl. Acad. Sci. USA 86:5728-5732; Alting-Mees et al. (1990) Strategies in Molecular Biology 3:1-9).

Within the context of the present invention, antibodies are understood to include various kinds of antibodies, including, but not necessarily limited to, naturally occurring antibodies, monoclonal antibodies, polyclonal antibodies, antibody fragments that retain antigen binding specificity (*e.g.*, Fab, and F(ab')₂) and recombinantly produced binding partners, single domain antibodies, hybrid antibodies, chimeric antibodies, single-chain antibodies, human antibodies, humanized antibodies, and the like. Generally, antibodies are understood to be reactive against a selected antigen of a cell if they bind with an affinity (association constant) of greater than or equal to 10⁷ M⁻¹. Antibodies against selected antigens for use with thecomplexes may be obtained from commercial sources.

Further description of the various kinds of antibodies of use as targeting ligands in the invention is provided herein, in particular, later in this *Compositions of the Invention* section.

The antigen-scaffold complexes of the present invention also employ targeting ligands other than an antibody or fragment thereof. For example, polypeptides, like antibodies, may have high specificity and epitope affinity for use as targeting ligands for targeted antigen-scaffold complexes. These may be small oligopeptides, having, for example, 5 to 10 amino acids, specific for a unique receptor sequences or larger polypeptides. Smaller peptides potentially have less inherent immunogenicity than nonhumanized murine antibodies. Peptides or peptide (nonpeptide) analogues of particular cell adhesion molecules, cytokines, selectins, cadhedrins, Ig superfamily and the like may be utilized for targeted delivery of the complexes.
"Non-peptide" moieties in general are those other than compounds which are simply polymers of amino acids, either gene encoded or non-gene encoded. Thus, "non-peptide ligands" are moieties which are commonly referred to as "small molecules" lacking in polymeric character and characterized by the requirement for a core structure other than a polymer of amino acids. The non-peptide ligands useful in the invention may be coupled to peptides or may include peptides coupled to portions of the ligand which are responsible for affinity to the target site, but it is the non-peptide regions of this ligand which account for its binding ability.

Carbohydrate-bearing lipids may be used for targeting of the complexes, as described, for example, in U.S. Pat. No. 4,310,505.

In some of the antigen-scaffold complexes of the invention, a targeting ligand is coupled, either directly or indirectly, to the surface of the complex. When the targeting ligand is coupled directly to the surface of a complex, the targeting ligand is either covalently or noncovalently attached to the surface of the antigen-scaffold complex. The coupling of the targeting ligand to the surface of the complex can be accomplished using techniques described herein and known in the art, including, but not limited to, direct covalent linkage, covalent conjugation via a crosslinker moiety and noncovalent conjugation (e.g., via a specific binding pair, via electrostatic bonding or via hydrophobic bonding).

When the targeting ligand is indirectly coupled to the surface of an antigen-scaffold complex, the targeting ligand is attached to a ligand linker and the linker is attached, either directly or indirectly, to a moiety on the surface of the complex. The targeting ligand is either covalently or non covalently attached to the ligand linker by techniques described herein and known in the art, including, but not limited to, direct covalent linkage, covalent conjugation via a crosslinker moiety (which may include a spacer arm) and noncovalent conjugation (e.g., via a specific binding pair (e.g., biotin and avidin), via electrostatic bonding or via hydrophobic bonding). The ligand linker is either directly or indirectly and either covalently or noncovalently attached to a moiety on the surface of an antigen-scaffold complex by techniques described herein and known in the art, including, but not limited to, direct covalent linkage, covalent conjugation via a crosslinker moiety (which may include a spacer arm), noncovalent conjugation via a specific binding pair (*e.g*., via a specific binding pair (*e.g*., biotin and avidin), via electrostatic bonding or via hydrophobic bonding).

Avidin-biotin interactions are useful, noncovalent targeting systems that have been incorporated into many biological and analytical systems and selected in vivo applications. Avidin has a high affinity for biotin (about 10⁻¹⁵ M) facilitating rapid and stable binding under physiological conditions. For example, the targeting ligand is attached to the surface of an antigen-scaffold complex through a linker comprised of a specific binding pair such as biotin and avidin or streptavidin. A biotin group can be attached, for example, to a moiety on the surface of an antigen-scaffold complex and avidin or streptavidin incorporated into or attached onto the targeting ligand. Alternatively, a biotin group can be attached to the targeting ligand and avidin or streptavidin attached to the surface of an antigen-scaffold complex. In either case, labeling one component with biotin and the other component with avidin or streptavidin allows for the formation of a non-covalently bound complex in which the targeting cell ligand is coupled to a biotin-(strept)avidin linker which is coupled to an antigen-scaffold complex. Methods and techniques for attaching biotin, avidin and streptavidin to molecules and cells are well known in the art. See, for example, O'Shannessey et al. (1984) *Supra*; O'Shannessy et al. (1985) *Supra.*

Alternatively, some targeted systems utilizing this approach are administered in two or three steps, depending on the formulation. Typically in these systems, a biotinylated ligand, such as a monoclonal antibody, is administered first and "pretargeted" to the unique molecular epitopes. Next, avidin is administered, which binds to the biotin moiety of the "pretargeted" ligand. Finally, the biotinylated antigen-scaffold complex is added and binds to the unoccupied biotin-binding sites remaining on the avidin thereby completing the ligand-avidin-complex "sandwich." The avidin-biotin approach can avoid accelerated, premature clearance of targeted agents by the reticuloendothelial system secondary to the presence of surface antibody. Additionally, avidin, with four, independent biotin binding sites provides signal amplification and improves detection sensitivity.

As used herein, the term "biotinylated" with respect to coupling to a biotin agent is intended to include biotin, biocytin and other biotin derivatives and analogs such as biotin amido caproate N-hydroxysuccinimide ester, biotin 4-amidobenzoic acid, biotinamide caproyl hydrazide and other biotin derivatives and conjugates. Other derivatives include biotin-dextran, biotin-disulfide N-hydroxysuccinimide ester, biotin-6 amido quinoline, biotin hydrazide, d-biotin-N hydroxysuccinimide ester, biotin maleimide, d-biotin p-nitrophenyl ester, biotinylated nucleotides and biotinylated amino acids such as N, epsilon-biotinyl-1-lysine. The term "avidinized" with respect to coupling to an avidin agent is intended to include avidin, streptavidin and other avidin analogs such as streptavidin or avidin conjugates, highly purified and fractionated species of avidin or streptavidin, and non-amino acid or partial-amino acid variants, recombinant or chemically synthesized avidin.

In some embodiments, the ligand linker can comprise at least one antibody, or the antigen binding portion thereof. An antibody that serves as a ligand linker can bind both the targeting ligand and a moiety or antigen on the antigen-scaffold complex. A ligand linker could comprise more than one antibody since one antibody could bind both the targeting ligand and a second antibody, and the second antibody could then bind a moiety on the antigen-scaffold complex.

Non-covalent associations can also occur through ionic interactions involving a targeting ligand and residues within a moiety on the surface of the antigen-scaffold complex. Non-covalent associations can also occur through ionic interactions involving a targeting ligand and residues within a ligand linker, such as charged amino acids, or through the use of a linker portion comprising charged residues that can interact with both the targeting ligand and the antigen-scaffold complex. For example, non-covalent conjugation can occur between a generally negatively-charged targeting ligand or moiety on an antigen-scaffold complex and positively-charged amino acid residues of a linker, *e.g.*, polylysine, polyarginine and polyhistidine residues.

Covalent conjugation of the targeting ligand to the ligand linker or the ligand linker to the moiety on the antigen-scaffold complex may be effected in any number of ways, typically involving one or more crosslinking agents and functional groups on the targeting ligand, linker molecule and/or the moiety on the antigen-scaffold complex.

Targeting ligands that are polypeptides will contain amino acid side chain moieties containing functional groups such as amino, carboxyl, or sulfhydryl groups that will serve as sites for coupling the targeting ligand to the ligand linker. Residues that have such functional groups may be added to the targeting ligand if the targeting ligand does not already contain these groups. Such residues may be incorporated by solid phase synthesis techniques or recombinant techniques, both of which are well known in the peptide synthesis arts. In the case of targeting ligands that are carbohydrate or lipid, functional amino and sulfhydryl groups may be incorporated therein by conventional chemistry. For instance, primary amino groups may be incorporated by reaction with ethylenediamine in the presence of sodium cyanoborohydride and sulfhydryls may be introduced by reaction of cysteamine dihydrochloride followed by reduction with a standard disulfide reducing agent. In a similar fashion, the linker molecule or the moiety on the antigen-scaffold complex may also be derivatized to contain functional groups if it does not already possess appropriate functional groups.

Hydrophilic linkers of variable lengths are useful for connecting peptides or other bioactive molecules to linker molecules. Suitable linkers include linear oligomers or polymers of ethyleneglycol. Such linkers include linkers with the formula R₁S(CH₂CH₂O)ₙCH₂CH₂O(CH₂)ₘCO₂R₂ wherein n=0-200, m=1 or 2, R₁ =H or a protecting group such as trityl, R₂ =H or alkyl or aryl, *e.g.*, 4-nitrophenyl ester. These linkers are useful in connecting a molecule containing a thiol reactive group such as haloaceyl, maleiamide, etc., via a thioether to a second molecule which contains an amino group via an amide bond. These linkers are generally flexible with regard to the order of attachment, *i.e.*, the thioether can be formed first or last.

Targeting ligands may be chemically attached to the surface of the antigen-scaffold complex by a variety of methods depending upon the nature of the complex. Conjugations maybe performed before or after the antigen-scaffold complex is created depending upon the ligand employed. Direct chemical conjugation of ligands to proteinaceous agents often take advantage of numerous amino-groups (e.g. lysine) inherently present within the surface. Alternatively, functionally active chemical groups such as pyridyldithiopropionate, maleimide or aldehyde may be incorporated into the surface as chemical "hooks" for ligand conjugation after the particles are formed. Another common post-processing approach is to activate surface carboxylates with carbodiimide prior to ligand addition. The selected covalent linking strategy is primarily determines by the chemical nature of the ligand. Antibodies and other large proteins may denature under harsh processing conditions; whereas, the bioactivity of carbohydrates, short peptides, aptamers, drugs or peptidomimetics often can be preserved. To ensure high ligand binding integrity and maximize targeted complex avidity flexible polymer spacer arms, e.g. polyethylene glycol or simple caproate bridges, can be inserted between an activated complex functional group and the targeting ligand. These extensions can be 10 nm or longer and minimize interference of ligand binding by particle surface interactions.

In addition to that described elsewhere herein, following is further description of the various kinds of antibodies appropriate for use as targeting ligands in and/or with the antigen-scaffold complexes of the invention.

Bivalent F(ab')₂ and monovalent F(ab) fragments can be used as ligands and these are derived from selective cleavage of the whole antibody by pepsin or papain digestion, respectively. Antibodies can be fragmented using conventional techniques and the fragments (including "Fab" fragments) screened for utility in the same manner as described above for whole antibodies. The "Fab" region refers to those portions of the heavy and light chains which are roughly equivalent, or analogous, to the sequences which comprise the branch portion of the heavy and light chains, and which have been shown to exhibit immunological binding to a specified antigen, but which lack the effector Fc portion. "Fab" includes aggregates of one heavy and one light chain (commonly known as Fab'), as well as tetramers containing the 2H and 2L chains (referred to as F(ab)₂), which are capable of selectively reacting with a designated antigen or antigen family. Methods of producing Fab fragments of antibodies are known within the art and include, for example, proteolysis, and synthesis by recombinant techniques. For example, F(ab')₂ fragments can be generated by treating antibody with pepsin. The resulting F(ab')₂ fragment can be treated to reduce disulfide bridges to produce Fab' fragments. "Fab" antibodies maybe divided into subsets analogous to those described herein, *i.e.*, "hybrid Fab", "chimeric Fab", and "altered Fab". Elimination of the Fc region greatly diminishes the immunogenicity of the molecule, diminishes nonspecific liver uptake secondary to bound carbohydrate, and reduces complement activation and resultant antibody-dependent cellular toxicity. Complement fixation and associated cellular cytotoxicity can be detrimental when the targeted cell or tissue must be preserved.

Most monoclonal antibodies are of murine origin and are inherently immunogenic to varying extents in other species. Humanization of murine antibodies through genetic engineering has lead to development of chimeric ligands with improved biocompatibility and longer circulatory half-lives. Antibodies used in the invention include those that have been humanized or made more compatible with the individual to which they will be administered. In some cases, the binding affinity of recombinant antibodies to targeted molecular epitopes can be improved with selective site-directed mutagenesis of the binding idiotype. Methods and techniques for such genetic engineering of antibody molecules are known in the art. By "humanized" is meant alteration of the amino acid sequence of an antibody so that fewer antibodies and/or immune responses are elicited against the humanized antibody when it is administered to a human. For the use of the antibody in a mammal other than a human, an antibody may be converted to that species format.

Phage display techniques may be used to produce recombinant human monoclonal antibody fragments against a large range of different antigens without involving antibody-producing animals. In general, cloning creates large genetic libraries of corresponding DNA (cDNA) chains deducted and synthesized by means of the enzyme "reverse transcriptase" from total messenger RNA (mRNA) of human B lymphocytes. By way of example, immunoglobulin cDNA chains are amplified by polymerase chain reaction (PCR) and light and heavy chains specific for a given antigen are introduced into a phagemid vector. Transfection of this phagemid vector into the appropriate bacteria results in the expression of as scFv immunoglobulin molecule on the surface of the bacteriophage. Bacteriophages expressing specific immunoglobulin are selected by repeated immunoadsorption/phage multiplication cycles against desired antigens (e.g., proteins, peptides, nuclear acids, and sugars). Bacteriophages strictly specific to the target antigen are introduced into an appropriate vector, (e.g., E. coli, yeast, cells) and amplified by fermentation to produce large amounts of human antibody fragments, generally with structures very similar to natural antibodies. Phage display techniques are known in the art and have permitted the production of unique ligands for targeting and therapeutic applications.

Polyclonal antibodies against selected antigens may be readily generated by one of ordinary skill in the art from a variety of warm-blooded animals such as horses, cows, various fowl, rabbits, mice, or rats. In some cases, human polyclonal antibodies against selected antigens may be purified from human sources.

As used herein, a "single domain antibody" (dAb) is an antibody which is comprised of a V_{H} domain, which reacts immunologically with a designated antigen. A dAb does not contain a V_{L} domain, but may contain other antigen binding domains known to exist in antibodies, for example, the kappa and lambda domains. Methods for preparing dAbs are known in the art. See, for example, Ward et al. (1989) Nature 341:544-546. Antibodies may also be comprised of V_{H} and V_{L} domains, as well as other known antigen binding domains. Examples of these types of antibodies and methods for their preparation are known in the art (see, *e.g.*, U.S. Pat. No. 4,816,467).

Further exemplary antibodies include "univalent antibodies", which are aggregates comprised of a heavy chain/light chain dimer bound to the Fc (*i.e.*, constant) region of a second heavy chain. This type of antibody generally escapes antigenic modulation. See, *e.g.*, Glennie et al. (1982) Nature 295:712-714.

"hybrid antibodies" are antibodies wherein one pair of heavy and light chains is homologous to those in a first antibody, while the other pair of heavy and light chains is homologous to those in a different second antibody. Typically, each of these two pairs will bind different epitopes, particularly on different antigens. This results in the property of "divalence", *i.e.*, the ability to bind two antigens simultaneously. Such hybrids may also be formed using chimeric chains, as set forth herein.

The invention also encompasses "altered antibodies", which refers to antibodies in which the naturally occurring amino acid sequence in a vertebrate antibody has been varied. Utilizing recombinant DNA techniques, antibodies can be redesigned to obtain desired characteristics. The possible variations are many, and range from the changing of one or more amino acids to the complete redesign of a region, for example, the constant region. Changes in the variable region may be made to alter antigen binding characteristics. The antibody may also be engineered to aid the specific delivery of a complex to a specific cell or tissue site. The desired alterations may be made by known techniques in molecular biology, *e.g.*, recombinant techniques, site directed mutagenesis, and other techniques.

"Chimeric antibodies", are antibodies in which the heavy and/or light chains are fusion proteins. Typically the constant domain of the chains is from one particular species and/or class, and the variable domains are from a different species and/or class. The invention includes chimeric antibody derivatives, *i*.*e*., antibody molecules that combine a non-human animal variable region and a human constant region. Chimeric antibody molecules can include, for example, the antigen binding domain from an antibody of a mouse, rat, or other species, with human constant regions. A variety of approaches for making chimeric antibodies have been described and can be used to make chimeric antibodies containing the immunoglobulin variable region which recognizes selected antigens on the surface of targeted cells and/or tissues. See, for example, Morison et al. (1985) Proc. Natl. Acad. Sci. U.S.A. 81:6851; Takeda et al. (1985) Nature 314:452; U.S. Pat. Nos. 4,816,567 and 4,816,397; European Patent Publications EP171496 and EP173494; United Kingdom patent GB 2177096B.

Bispecific antibodies may contain a variable region of an anti-target site antibody and a variable region specific for at least one antigen on the antigen-scaffold complex. In other cases, bispecific antibodies may contain a variable region of an anti-target site antibody and a variable region specific for a ligand linker molecule. Bispecific antibodies may be obtained forming hybrid hybridomas, for example by somatic hybridization. Hybrid hybridomas may be prepared using the procedures known in the art such as those disclosed in Staerz et al. (1986, Proc. Natl. Acad. Sci. U.S.A. 83:1453) and Staerz et al. (1986, Immunology Today 7:241). Somatic hybridization includes fusion of two established hybridomas generating a quadroma (Milstein et al. (1983) Nature 305:537-540) or fusion of one established hybridoma with lymphocytes derived from a mouse immunized with a second antigen generating a trioma (Nolan et al. (1990) Biochem. Biophys. Acta 1040:1-11). Hybrid hybridomas are selected by making each hybridoma cell line resistant to a specific drug-resistant marker (De Lau et al. (1989) J. Immunol. Methods 117:1-8), or by labeling each hybridoma with a different fluorochrome and sorting out the heterofluorescent cells (Karawajew et al. (1987) J. Immunol, Methods 96:265-270).

Bispecific antibodies may also be constructed by chemical means using procedures such as those described by Staerz et al. (1985) Nature 314:628 and Perez et al. (1985) Nature 316:354. Chemical conjugation may be based, for example, on the use of homo-and heterobifunctional reagents with E-amino groups or hinge region thiol groups. Homobifunctional reagents such as 5,5'-dithiobis(2-nitrobenzoic acid) (DNTB) generate disulfide bonds between the two Fabs, and 0-phenylenedimaleimide (O-PDM) generate thioether bonds between the two Fabs (Brenner et al. (1985) Cell 40:183-190, Glennie et al. (1987) J. Immunol. 139:2367-2375). Heterobifunctional reagents such as N-succinimidyl-3-(2-pyridylditio) propionate (SPDP) combine exposed amino groups of antibodies and Fab fragnents, regardless of class or isotype (Van Dijk et al. (1989) Int. J. Cancer 44:738-743).

Bifunctional antibodies may also be prepared by genetic engineering techniques. Genetic engineering involves the use of recombinant DNA based technology to ligate sequences of DNA encoding specific fragments of antibodies into plasmids, and expressing the recombinant protein. Bispecific antibodies can also be made as a single covalent structure by combining two single chains Fv (scFv) fragments using linkers (Winter et al. (1991) Nature 349:293-299); as leucine zippers coexpressing sequences derived from the transcription factors fos and jun (Kostelny et al. (1992) J. Immunol. 148:1547-1553); as helix-turn-helix coexpressing an interaction domain of p53 (Rheinnecker et al. (1996) J. Immunol. 157:2989-2997), or as diabodies (Holliger et al. (1993) Proc. Natl. Acad. Sci. U.S.A. 90:6444-6448).

### Kits of the invention

The invention also provides kits. In certain embodiments, the kits of the invention comprise one or more containers comprising antigen-scaffold complexes of the invention. In other embodiments, the kits of the invention comprise one or more containers comprising antigens for use in the complexes and/or one or more containers comprising reagents for scaffold molecules for use in the complexes. The kits may further comprise a suitable set of instructions, generally written instructions, relating to the use of the antigen-scaffold complexes for the intended treatment (*e.g.*, immunomodulation, ameliorating symptoms of an infectious disease, ameliorating symptoms of a cancer, or ameliorating an autoimmune disorder).

The kits may comprise antigen-scaffold complexes, or components to make antigen-scaffold complexes, packaged in any convenient, appropriate packaging. For example, if the antigen-scaffold complex is a dry formulation (*e.g.*, freeze dried or a dry powder), a vial with a resilient stopper is normally used, so that the antigen-scaffold complex may be easily resuspended by injecting fluid through the resilient stopper. Ampoules with non-resilient, removable closures (*e.g.*, sealed glass) or resilient stoppers are most conveniently used for liquid formulations of antigen-scaffold complexes.

The instructions relating to the use of antigen-scaffold complexes generally include information as to dosage, dosing schedule, and route of administration for the intended treatment. The containers of antigen-scaffold complexes may be unit doses, bulk packages (*e.g.*, multi-dose packages) or sub-unit doses. Instructions supplied in the kits of the invention are typically written instructions on a label or package insert (*e.g.*, a paper sheet included in the kit), but machine-readable instructions (*e.g.*, instructions carried on a magnetic or optical storage disk) are also acceptable.

### Methods of use of the compositions

The invention provides methods of modulating an immune response in an individual comprising administering to the individual an antigen-scaffold complex and/or cells which have been treated with an antigen-scaffold complex as described herein. The particular method and antigen-scaffold complex used in the method will depend on the need of the recipient individual and the type of immune modulation desired (e.g., enhancement or suppression).

A number of individuals are suitable for receiving the antigen-scaffold complex(es) described herein. Preferably the individual is a mammal and more preferably, but not necessarily, the individual is human. As used herein, and as well-understood in the art, "treatment" is an approach for obtaining beneficial or desired results, including clinical results. For purposes of this invention, beneficial or desired clinical results include, but are not limited to, alleviation or amelioration of one or more symptoms, diminishment of extent of disease, stabilized (*i.e.*, not worsening) state of disease, preventing spread of disease, preventing or delaying or slowing of disease progression, amelioration or palliation of the disease state, and remission (whether partial or total), whether detectable or undetectable. "Treatment" can also mean prolonging survival as compared to expected survival if not receiving treatment. "Palliating" a disease or disorder means that the extent and/or undesirable clinical manifestations of a disorder or a disease state are lessened and/or time course of the progression is slowed or lengthened, as compared to not treating the disorder. Further, palliation does not necessarily occur by administration of one dose, but often occurs upon administration of a series of doses. Thus, an amount sufficient to palliate a response or disorder may be administered in one or more administrations.

In certain embodiments, the individual subject to the immunomodulatory methods of the invention is an individual receiving the antigen-scaffold complex as a vaccine. The vaccine may be a prophylactic vaccine or a therapeutic vaccine. A prophylactic vaccine comprises antigen-scaffold complexes in which the antigens are associated with a disorder for which the individual may be at risk (e.g., HIV antigens as a vaccine for prevention of HIV-associated disorders; *M. tuberculosis* antigens as a vaccine for prevention of tuberculosis). Therapeutic vaccines comprise antigen-scaffold complexes in which the antigens are associated with a particular disorder affecting the individual, such as *M. tuberculosis* surface antigens in tuberculosis patients or tumor associated antigens in cancer patients. Administration of the antigen-scaffold complex as a vaccine results in an immune response to the antigens and cells expressing the antigens, in particular a T cell immune response.

In the case of therapeutic vaccines, administration of antigen-scaffold complexes also results in amelioration of one or more symptoms of the disorder which the vaccine is intended to treat. As will be apparent to one of skill in the art, the exact symptoms and manner of their improvement will depend on the disorder sought to be treated. For example, where the therapeutic vaccine is for hepatitis, antigen-scaffold complex vaccine results in reduction of one or more symptoms of hepatitis infection (e.g., jaundice, fatigue, abdominal pain, viremia, portal hypertension, cirrhosis and/or blood levels of liver enzymes).

Accordingly, embodiments of the invention relate to therapy of individuals having a pre-existing disease or disorder, such as cancer or an infectious disease. Cancer is an attractive target for the antigen-scaffold complexes of the invention because most cancers express tumor-associated and/or tumor specific antigens. Stimulation and/or enhancement of a T cell response against tumor cells results in direct and/or bystander killing of tumor cells by the immune system, leading to a reduction in cancer cells and a reduction in symptoms. Administration of an antigen-scaffold complex to an individual having cancer results in stimulation of an immune response, particularly a T cell response, against the tumor cells. Such an immune response can kill tumor cells, either by direct action of cellular immune system cells (e.g., CTLs) or components of the humoral immune system, or by bystander effects on cells proximal to cells targeted by the immune system.

Cancers which are responsive to antigen-scaffold complexes of the invention or to APCs sensitized to the antigen scaffold complexes described below include, but are not limited to human sarcomas and carcinomas, e.g., melanoma, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, neuroblastoma, retinoblastoma; leukemias, e.g., acute lymphocytic leukemia and acute myelocytic leukemia (myeloblastic, promyelocytic, myelomonocytic, monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenstrom's macroglobulinemia, and heavy chain disease.

Therapeutic vaccines and therapy in accordance with the invention is also useful for individuals with infectious diseases, particularly infectious diseases which are resistant to humoral immune responses (e.g., diseases caused by mycobacterial infections and intracellular pathogens). Antigen-scaffold complex therapy may be used for the treatment of infectious diseases caused by cellular pathogens (e.g., bacteria or protozoans) or by subcellular pathogens (e.g., viruses). Antigen-scaffold complex therapy may be administered to individuals suffering from mycobacterial diseases such as tuberculosis (e.g., M. tuberculosis and/or M. bovis infections), leprosy (i.e., M. leprae infections), or M. marinum or M. ulcerans infections. Antigen-scaffold complex therapy is also useful for the treatment of viral infections, including infections by human immunodeficiency virus (HIV), influenza virus, respiratory syncytial virus, hepatitis virus B, hepatitis virus C, herpes viruses, particularly herpes simplex viruses, and papilloma viruses. Diseases caused by intracellular parasites such as malaria (e.g., infection by Plasmodium vivax, P. ovale, P. falciparum and/or P. malariae), leishmaniasis (e.g., infection by Leishmania donovani, L. tropica, L. mexicana, L. braziliensis, L. peruviana, L. infantum, L. chagasi, and/or L. aethiopica), and toxoplasmosis (i.e., infection by Toxoplasmosis gondii) also benefit from antigen-scaffold complex therapy. Antigen-scaffold complex therapy is also useful for treatment of parasitic diseases such as schistosomiasis (i.e., infection by blood flukes of the genus Schistosoma such as S. haematobium, S. mansoni, S. japonicum, and S. mekongi) and clonorchiasis (i.e., infection by Clonorchis sinensis). Administration of antigen-scaffold complex(es) to an individual suffering from an infectious disease results in an amelioration of symptoms of the infectious disease.

In certain embodiments, the individual subject to immunomodulatory methods of the invention is an individual receiving cells which have been treated *ex vivo* with the antigen-scaffold complex described herein. These embodiments comprise *ex vivo* treatment of cells, particularly PBMCs, more particularly dendritic cells, with the antigen-scaffold complex(es) and administration of the treated cells to the individual where they serve to enhance the immune response to the antigen. Preferably, the population of cells treated with the complex will be depleted of CD4+/CD25+ T cells prior to administration to the individual. Removal of the CD4+/CD25+ T cells prior to administration to the recipient individual reduces or inhibits antigen tolerance in the recipient and any resulting suppression of the immune response. Onizuka et al. (1999) Cancer Res. 59:3128-3133; Shimizu et al. (1999) J. Immunol. 163:5211-5218.

The cells for *ex vivo* methods are collected from an individual and put in culture conditions as needed. For example, T cells and/or dendritic cells can be obtained from peripheral blood mononuclear cells (PBMCs), lymph node, spleen and/or bone marrow. Methods for collecting and culturing such cells are known in the art. Methods for generating large numbers of monocyte-derived dendritic cells, including methods for isolating and maturation, are described, for example, by Thumer et al. (1999) J. Immunol. Methods 223:1-15 and Pullarkat et al. (2002) J. Immunol. Methods 267:173-183. APCs, e.g., dendritic cells, are sensitized to the antigens of the complex through incubation with the antigen-scaffold complexes in vitro.

Individuals for whom the *ex vivo* methods of the invention are appropriate are the same types of individuals for whom the *in vivo* vaccine methods are appropriate, i.e., individuals at risk of or afflicted with disorders that would benefit from an enhanced T cell immune response, e.g., those at risk of or afflicted with infectious disease and/or cancer.

Whether the antigen-scaffold complexes are used with *in vivo*, *ex vivo* or *in vitro* methods, in some embodiments of the methods, the complexes administered to the individual and/or added to the cells contain antigen peptides with epitopes that are specifically presented by the MHC class I or class II molecules of the recipient individual and/or cells. For example, for a recipient cell population with alleles HLA-A2, HLA-A3, HLA-DR1, HLA-DR4, antigen-scaffold complexes comprising antigen peptides that are presented by these particular MHC molecules are used.

Whether the antigen-scaffold complexes are used with *in vivo*, *ex vivo* or *in vitro* methods, in some embodiments, the complexes are used in conjunction with as adjuvant or other immunostimulatory agents, such as cytokines and chemokines. "Adjuvant" refers to a substance which, when added to an immunogenic agent such as antigen, nonspecifically enhances or potentiates an immune response to the agent in the recipient host upon exposure to the mixture. Suitable immunostimulatory agents include, but are not limited to, immunostimulatory polynucleotides, Flt-3 ligand, CD40 ligand, OX40 (CD134), Trance/RankL, TNFα, IL-1, IL-2 and CCR7 plasmid. As known in the art and described herein, immunostimulatory polynucleotides containing an unmethylated CpG dinucleotide are useful as a component of the complex to target the complex to dendritic cells and other cells of the immune system. Such immunostimulatory polynucleotides are also of use as an adjuvant when co-administered with the antigen-scaffold complex, not necessarily as a component of the complex. Since D-type immunostimulatory oligonucleotides also stimulate monocytes to differentiate into mature dendritic cells (as described herein), these molecules are of particular use with the complexes of the invention. Klinman et al. (2002), *Supra.* Adjuvants are known in the art and include, but are not limited to, oil-in-water emulsions, water-in oil emulsions, alum (aluminum salts), liposomes and microparticles, including but not limited to, polystyrene, starch, polyphosphazene and polylactide/polyglycosides. Other suitable adjuvants also include, but are not limited to, MF59, DETOX™ (Ribi), squalene mixtures (SAF-1), muramyl peptide, saponin derivatives, mycobacterium cell wall preparations, monophosphoryl lipid A, mycolic acid derivatives, nonionic block copolymer surfactants, Quil A, cholera toxin B subunit, polyphosphazene and derivatives, and immunostimulating complexes (ISCOMs). The particular adjuvant or agent used will depend on the type of cell to which the complex is targeted and the type of immune response desired. For example, dendritic cells can be incubated with antigen-scaffold complex(es) and with CD40L and, eventually, with T cells. CD40L (CD40 ligand, a member of the TNF family) binds to CD40 on antigen presenting cells (e.g., dendritic cells) which then transmits activating signals to the T cell and activates the APC to express co-stimulatory B7 molecules, thus further stimulating T cell proliferation. Accordingly, the addition of CD40L to the antigen-scaffold complex treated cells provides co-stimulatory signals to the activated T cells resulting in clonal expansion and differentiation of the T cells.

In certain embodiments, the methods of the invention are directed to treating is an individual suffering from an autoimmune disorder or in need of specific immune suppression (e.g., transplant recipient). These methods are based on the ability of T regulatory cells (CD4+/CD25+) to suppress the activity of autoreactive cells and thus, suppress or inhibit an autoimmune response or disease or suppress transplant rejection. Without wishing to be bound by theory, it is believed that such regulatory T cells work through the release of cytokines, such as transforming growth factor-β (TGF-β), which specifically inhibit the autoreactive T cells. See, for example, Jiang et al. (1992) Science 256:1213; Miller et al. (1992) Proc. Natl. Acad. Sci. USA 89:421-425.

These embodiments comprise *ex vivo* treatment of cells, particularly PBMCs, more particularly dendritic cells, with the antigen-scaffold complex(es) and subsequent recovery of CD4+/CD25+ T cells from the treated cell population. These activated T regulatory cells are then administered to the individual where they serve to suppress autoimmune responses in the individual. In some embodiments, the cells used in this method are isolated from the individual to whom they eventually will be re-admisistered. In other embodiments, the cells will be allogeneic to the recipient individual. In some embodiments, cells treated with the antigen-scaffold complex will also be treated with an adjuvant- Recovery of the CD4+/CD25+ T cells from the treated population can occur using methods known in the art, including, but not limited to, positive selection of the cells with agents that specifically react with CD4+/CD25+ T cells and negative selection to remove CD4+/CD8+ cells. Techniques and reagents for positive and negative cell selection are known in the art and available commercially, for example from Miltenyi Biotech.

Autoimmune associated disorders for which the antigen-scaffold complexes and cell treated with the antigen-scaffold complexes of the invention may be employed to relieve the symptoms of, treat or prevent the occurrence or reoccurrence of include, for example, MS, RA, Sjogren syndrome, scleroderma, polymyositis, dermatomyositis, systemic lupus erythematosus, juvenile rheumatoid arthritis, ankylosing spondylitis, MG, bullous pemphigoid, pemphigus, glomerulonephritis, Goodpasture's syndrome, autoimmune hemolytic anemia, Hashimoto's disease, pernicious anemia, idiopathic thrombocytopenic purpura, Grave's disease, and Addison's disease, and the like.

In other embodiments, the invention is directed to methods of identifying ummmodominant MHC epitopes. After treating cells with the multi-antigen complexes of the invention, the antigen peptides associated with the MHC class I and class II molecules of the treated cells can be recovered and the peptides analyzed to identify the immunodominant MHC epitopes, the epitopes more frequently presented in the context of the MHC molecules of the particular cells. One way of identifying an antigen peptide containing a MHC epitope, when T cells are present, is to elute the peptide with an acid or base. The peptides associated with MHC molecules are present on antigen presenting cells and on the cells that are lysed by CTL. The eluted peptides are separated using a purification method such as HPLC, and individual fractions are tested for their MHC binding activity, e.g, the capacity to sensitize targets for CTL lysis. Another way to identify an antigen peptide is to cleave the MHC-antigen complex from the cells, elute the antigen from the MHC molecule and isolate the antigen with mass spectrometry. When a fraction has been identified as containing the MHC peptide, it is further purified and submitted to sequence analysis. The peptide sequence can also be determined using tandem mass spectrometry. A synthetic peptide can then be prepared and tested for MHC binding activity to corroborate that the correct sequence and peptide have been identified. Techniques for eluting and recovering peptides from MHC-pcptide complexes are described in the art, for example, in Falk et al. (1990) Nature 348:248-251, Elliot et al. (1990) Nature 348:195-197, Falk et al. (1991), *Supra.*

### Administration and assessment of the immune response

According to still another aspect of the invention, the compositions of the invention, including antigen-scaffold complexes, compositions comprising antigen-scaffold complexes and compositions comprising cells stimulated and/or generated using the methods of the invention, and mixtures thereof, are used in the preparation of medicaments, for treating the conditions described herein. These compositions of the invention are administered as pharmaceutically acceptable compositions. The antigen-scaffold complex can be administered in combination with other pharmaceutical and/or immunostimulatory agents, as described herein, and can be combined with a physiologically acceptable carrier. The compositions may be administered by any suitable means, including, but not limited to, intravenously, parenterally or locally. The compositions can be administered in a single dose by bolus injection or continuous infusion or in several doses over selected time intervals in order to titrate the dose.

In some embodiments, the antigen-scaffold complexes are administered in conjunction with a composition comprising free antigen and an adjuvant or other immunostimulatory agent. For example, the complexes are administered with an emulsion of free antigen peptides and an adjuvant. In such a case, the free antigen peptides may be the same mix of antigens used in the administered complex.

As used herein, "pharmaceutically acceptable excipient" includes any material which, when combined with an active ingredient of a composition, allows the ingredient to retain biological activity without causing disruptive reactions with the subject's immune system. Various pharmaceutically acceptable excipients are well known in the art.

Exemplary pharmaceutically acceptable excipients include sterile aqueous or nonaqueous solutions and suspensions. Examples include, but are not limited to, any of the standard pharmaceutical excipients such as a phosphate buffered saline solution, water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Intravenous vehicles include fluid and nutrient replenishers, electrolyte replenishers (such as those based on Ringer's dextrose), and the like. Compositions comprising such excipients are formulated by well known conventional methods (see: for example, Remington's Pharmaceutical Sciences, 18th Ed., Mack Publishing Co.).

As with all immunogenic compositions, the immunologically effective amounts and method of administration of the particular antigen-scaffold complex or cells treated with the antigen-scaffold complex can vary based on the individual, what condition is to be treated and other factors evident to one skilled in the art. Factors to be considered include the antigenicity, whether or not the antigen-scaffold complex will be administered with an adjuvant or immunostimulatory agent, route of administration and the number of immunizing doses to be administered, the stage and severity of disease being treated, the weight and general health of the recipient individual and the judgement of the prescribing physician. Such factors are known in the art and it is well within the skill of those in the art to make such determinations without undue experimentation. An "effective amount" or a "sufficient amount" of a substance is that amount sufficient to effect beneficial or desired results, including clinical results, and, as such, an "effective amount" depends upon the context in which it is being applied. An effective amount can be administered in one or more administrations.

A suitable dosage range is one that provides the desired modulation of immune response to the antigen. Generally, dosage is determined by the amount of antigen administered to the patients, rather than the overall quantity of antigen-scaffold complex. Useful dosage ranges of the antigen-scaffold complex, given in amounts of antigen delivered, may be, for example, from about any of the following: 0.01 µg to 1000 µg per dose, 0.1 µg to 100 µg per dose, and 1.0 µg to 10 µg per dose. Generally, dosage ranges for initial immunization (that is for therapeutic or prophylactic administration) are from about any of the following: 1.0 µg to 100 µg per dose, 1.0 µg to 50 µg per dose, 1.0 µg to 10 µg per dose, followed by boosting dosages of from about any of the following: 1.0 µg to 100 µg per dose, 1.0 µg to 50 µg per dose, 1.0 µg to 10 µg per dose, pursuant a boosting regimen over weeks to months depending upon the individual's response and condition by measuring, for example, CTL activity of cells circulating in the individual. Suitable volumes for parenteral administration are about 0.1 to 1.0 ml per injection site. The absolute amount given to each patient depends on pharmacological properties such as bioavailability, clearance rate and route of administration.

For the administration of *ex vivo* treated cells, typically, about 10⁶-10¹⁰ cells can be administered in a volume of 50 µl to 1 liter, 1 ml to 1 liter, 10 ml to 250 ml, 50 ml to 150, and typically 100 ml. The volume will depend upon, for example, the type of cell administered, the disorder treated and the route of administration.

Single or multiple administration of the compositions and/or cells can be carried out with dose levels and pattern being selected by the treating physician.

The effective amount and method of administration of the particular antigen-scaffold complex can vary based on the individual patient and the stage of the disease and other factors evident to one skilled in the art. The route(s) of administration useful in a particular application are apparent to one of skill in the art. Routes of administration include but are not limited to topical, dermal, transdermal, transmucosal, epidermal, parenteral, gastrointestinal, and naso-pharyngeal and pulmonary, including transbronchial and transalveolar. The absolute amount given to each patient depends on pharmacological properties such as bioavailability, clearance rate and route of administration.

As described herein, APCs and tissues with high concentration of APCs are preferred targets for the antigen-scaffold complex. Thus, administration of antigen-scaffold complex to mammalian skin and/or mucosa, where APCs are present in relatively high concentration, is preferred.

The present invention provides antigen-scaffold complexes suitable for topical application including, but not limited to, physiologically acceptable implants, ointments, creams, rinses and gels, Topical administration is, for instance, by a dressing or bandage having dispersed therein a delivery system, by direct administration of a delivery system into incisions or open wounds, or by transdermal administration device directed at a site of interest. Creams, rinses, gels or ointments having dispersed therein an antigen-scaffold complex are suitable for use as topical ointments.

Preferred routes of dermal administration are those which are least invasive. Preferred among these means are transdermal transmission, epidermal administration and subcutaneous injection. Of these means, epidermal administration is preferred for the greater concentrations of APCs expected to be in intradermal tissue.

Transdermal administration is accomplished by application of a cream, rinse, gel, etc. capable of allowing the antigen-scaffold complex to penetrate the skin and enter the blood stream. Compositions suitable for transdermal administration include, but are not limited to, pharmaceutically acceptable suspensions, oils, creams and ointments applied directly to the skin or incorporated into a protective carrier such as a transdermal device (so-called "patch"). Examples of suitable creams, ointments etc. can be found, for instance, in the Physician's Desk Reference. For transdermal transmission, iontophoresis is a suitable method. Iontophoretic transmission can be accomplished using commercially available patches which deliver their product continuously through unbroken skin for periods of several days or more. Use of this method allows for controlled transmission of pharmaceutical compositions in relatively great concentrations, permits infusion of combination drugs and allows for contemporaneous use of an absorption promoter.

For transdermal transmission, low-frequency ultrasonic delivery is also a suitable method. Mitragotri et al. (1995) Science 269:850-853. Application of low-frequency ultrasonic frequencies (about 1 MHz) allows the general controlled delivery of therapeutic compositions, including those of high molecular weight.

Epidermal administration essentially involves mechanically or chemically irritating the outermost layer of the epidermis sufficiently to provoke an immune response to the irritant. Specifically, the irritation should be sufficient to attract APCs to the site of irritation, An exemplary mechanical irritant means employs a multiplicity of very narrow diameter, short tines which can be used to irritate the skin and attract APCs to the site of irritation, to take up antigen-scaffold complex transferred from the end of the tines. For example, the MONO-VACC old tuberculin test manufactured by Pasteur Merieux of Lyon, France contains a device suitable for introduction of antigen-scaffold complexes.

Another suitable approach to epidermal administration of antigen-scaffold complex is by use of a chemical which irratates the outermost cells of the epidermis, thus provoking a sufficient immune response to attact APCs to the area. An example is a keratinolytic agent, such as the salicylic acid used in the commercially available topical depilatory creme sold by Noxema Corporation under the trademark NAIR. This approach can also be used to achieve epithelial administration in the mucosa. The chemical irritant can also be applied in conjunction with the mechanical irritant (as, for example, would occur if the MONO-VACC type tine were also coated with the chemical irritant). The antigen-scaffold complex can be suspended in a carrier which also contains the chemical irritant or coadministered therewith.

Parenteral routes of administration include but are not limited to electrical (iontophoresis) or direct injection such as direct injection into a central venous line, intravenous, intramuscular, intraperitoneal, intradermal, or subcutaneous injection. Formulations of antigen-scaffold complex suitable for parenteral administration are generally formulated in USP water or water for injection and may further comprise pH buffers, salts bulking agents, preservatives, and other pharmaceutically acceptable excipients. Antigen-scaffold complex for parenteral injection may be formulated in pharmaceutically acceptable sterile isotonic solutions such as saline and phosphate buffered saline for injection.

Gastrointestinal routes of administration include, but are not limited to, ingestion and rectal. The invention includes antigen-scaffold complex formulations suitable for gastrointestinal administration including, but not limited to, pharmaceutically acceptable powders, pills or liquids for ingestion and suppositories for rectal administration. As will be apparent to one of skill in the art, pills or suppositories will further comprise pharmaceutically acceptable solids, such as starch, to provide bulk for the composition.

Naso-pharyngeal and pulmonary administration include are accomplished by inhalation, and include delivery routes such as intranasal, transbronchial and transalveolar routes. The invention includes formulations of antigen-scaffold complex suitable for administration by inhalation including, but not limited to, liquid suspensions for forming aerosols as well as powder forms for dry powder inhalation delivery systems. Devices suitable for administration by inhalation of antigen-scaffold complex formulations include, but are not limited to, atomizers, vaporizers, nebulizers, and dry powder inhalation delivery devices.

Analysis (both qualitative and quantitative) of the immune response to antigen-scaffold complex or to the cells treated with the antigen-scaffold complex can be by any method known in the art, including, but not limited to, measuring activation of specific populations of lymphocytes such as CD4+ T cells or CD8+ CTLs, production of cytokines such as IFN-γ, IFN-α, IL-2, IL-4, IL-5, IL-10 or IL-12 and/or antigen-specific antibody production (including measuring specific antibody subclasses). Measurement of a T cell proliferative response can be performed for instance through measuring BrdU incorporation as known in the art. Measurement of numbers of specific types of lymphocytes such as CD4+ T cells can be achieved, for example, with fluorescence-activated cell sorting (FACS). Cytotoxicity and CTL assays, such as chromium release assays, can be performed as known in the art. Cytokine concentrations can be measured, for example, by ELISA. Methods for measuring specific antibody responses include enzyme-linked immunosorbent assays (ELISA and ELISPOT) and are well known in the art. These and other assays to evaluate the immune response to an immunogen are well known in the art. See, for example, Current Protocols in Immunology (1991, Coligan et al., eds.).

The term "co-administration" as used herein refers to the administration of at least two different substances sufficiently close in time to modulate an immune response. Preferably, co-administration refers to simultaneous administration of at least two different substances.

As used herein, an "individual" is a vertebrate, preferably a mammal, more preferably a human. Mammals include, but are not limited to, humans, farm animals, sport animals, rodents and pets.

As used herein, the singular form "a", "an", and "the" includes plural references unless indicated otherwise. For example, "a" target cell includes one or more target cells.

As used herein, the term "comprising" and its cognates are used in their inclusive sense; that is, equivalent to the term "including" and its corresponding cognates.

The examples offered herein are to illustrate but not to limit the invention.

### THE FOLLOWING ARE NOT CLAIMS. THEY ARE STATEMENTS WHICH DESCRIBE SOME EMBODIMENTS OF THE INVENTION

1. An antigen-scaffold complex comprising 15 or more different antigens, wherein the antigens each comprise at least one MHC class I or MHC class II epitope.
2. The antigen-scaffold complex of statement 1, wherein the complex comprises 100 or more different antigens.
3. The antigen-scaffold complex of statement 1, wherein the complex comprises the antigens coupled to a scaffold molecules.
4. The antigen-scaffold complex of statement 3, wherein the scaffold molecule comprises agarose, dextran or polylysine, or a derivative thereof.
5. The antigen-scaffold complex of any of statements 1-4, wherein the antigens are peptide antigens.
6. The antigen-scaffold complex of statement 5, wherein the antigen peptides comprising at least one MHC class I epitope further comprise additional hydrophobic amino acid residues or additional basic amino acid residues at the carboxy termini of the antigen peptides.
7. The antigen-scaffold complex of statement 5, wherein the antigen peptides comprising at least one MHC class I epitope further comprise about 1-3 Additional lysine residues.
8. The antigen-scaffold complex of statement 5, wherein the antigen peptides further comprise an additional alanine-proline sequence.
9. The antigen-scaffold complex of statement 5, wherein the antigen peptides are coupled to the scaffold molecule with linkers comprising proteolytic substrates.
10. The antigen-scaffold complex of statement 9, wherein the linkers comprise substrates for proteosome or endosomal proteases.
11. The antigen-scaffold complex of statement 1, wherein the complex comprises antigens encapsulated in a liposome or coupled to the surface of a microcarrier particle.
12. The antigen-scaffold complex of statement 1 or statement 11, wherein the complex further comprises a targeting ligand.
13. The antigen-scaffold complex of statement 12, wherein the targeting ligand binds a molecule on the surface of a dendritic cell.
14. The antigen-scaffold complex of statement 13, wherein the targeting ligand binds to a molecule selected from the group consisting of langerin, DEC-205, DC-SIGN, TLR-3 ligand and TLR-9 ligand.
15. The antigen-scaffold complex of statement 1, further comprising a D-type immunostimulatory oligonucleotide.
16. The antigen-scaffold complex of statement 1, wherein the complex comprises antigen peptides from MAGE-A3 or from tyrosinase.
17. A composition comprising a multiplicity of antigen-scaffold complexes, wherein the composition comprises 15 or more different antigens coupled to the complexes, wherein the antigens each comprise at least one MHC class I or MHC class II epitope.
18. A composition of statement 17, wherein the composition comprises 100 or more different antigens.
19. A composition of statement 17, further comprising an adjuvant or immunostimulatory agent.
20. A composition comprising the antigen-scaffold complex of any of statements 1-16 and a pharmaceutically acceptable excipient.
21. A method of stimulating a T cell immune response in an individual comprising administering a composition according to statement 20.
22. A method of treating an infectious disease in an individual comprising administering an antigen-scaffold complex of any of statements 1-15, wherein the antigens are antigens of the infectious agent and wherein the complex is administered in an amount effective to stimulate a T cell response to the infectious agent.
23. A method of vaccinating an individual against an infectious agent comprising administering an antigen-scaffold complex of any of statements 1-15, wherein the antigens are antigens of the infectious agent and wherein the complex is administered in an amount effective to stimulate a T cell response to the infectious agent.
24. A method of vaccinating an individual against a cancer comprising administering an antigen-scaffold complex of any of statements 1-16, wherein the antigens are expressed on the cancer cells and wherein the complex is administered in an amount effective to stimulate a T cell response to the cancer cells.
25. A method of treating cancer in an individual comprising administering an antigen-scaffold complex of any of statements 1-16, wherein the antigens are expressed on the cancer cells and wherein the complex is administered in an amount effective to stimulate a T cell response to the cancer cells.
26. A method of treating cancer in an individual comprising:
   a) isolating a population of cells from the individual which includes dendritic cells or dendritic precursor cells;
   b) culturing the population of cells to stimulate proliferation and maturation of dendritic cells;
   c) contacting the dendritic cells with an antigen-scaffold complex of any of statements 1-16, wherein the antigens are expressed on the cancer cells;
   d) removing CD25+/CD4+ cells from the population of cells; and
   e) administering the population of cells without CD25+/CD4+ cells to the individual in an amount effective to stimulate a T cell response to the cancer cells.
27. A method of treating an autoimmune disorder in an individual comprising:
   a) isolating a population of cells from the individuals which includes dendritic cells or dendritic precursor cells;
   b) culturing the population of cells to stimulate proliferation and maturation of dendritic cells;
   c) contacting the dendritic cells with an antigen-scaffold complex according to any of statements 1-14, wherein the antigens are autoantigens;
   d) collecting CD25+/CD4+ cells from the population of cells; and
   e) administering the CD25+/CD4+ cells to the individual in an amount effective to suppress a symptom of the autoimmune disorder.
28. A method of identifying immunodominant antigen epitope in a population of antigens comprising:
   a) contacting a population of dendritic cells with antigen-scaffold complexes of any of statements 1-15:
   b) collecting the population of cells;
   c) eluting antigens from the surface of the population of cells; and
   d) purifying the eluted antigens.

## Claims

1. A multivalent scaffold molecule containing multiple sites that allow for covalent attachment of antigens and cytokines or chemokines and to which a multiplicity of antigens and cytokines or chemokines are bound, and wherein at least some of the antigens are different from each other, and wherein the antigens collectively provide both MHC class I and class II epitopes.

2. The scaffold molecule of claim 1, wherein the antigens are peptide antigens.

3. The scaffold molecule of claim 2, wherein the peptide antigens are coupled to the scaffold molecule with linkers comprising proteolytic substrates.

4. The scaffold molecule of claim 3, wherein the linkers comprise substrates for proteosome or endosomal proteases.

5. The scaffold molecule of any of claims 1-4, which comprises a targeting ligand.

6. The scaffold molecule of claim 5, wherein the targeting ligand binds a molecule on the surface of a dendritic cell.

7. A composition comprising the scaffold molecule of any of claims 1-8 and a pharmaceutically acceptable excipient.

8. The composition of claim 7 for use in a method of stimulating a T cell immune response in an individual.

9. The composition of claim 7 for use in a method to treat or vaccinate against an infectious disease in an individual.

10. The composition of claim 7 for use in a method to treat or vaccinate an individual against a cancer.

11. The scaffold molecule of any of claims 1-6 for use in a method of treating cancer in an individual comprising:
a) culturing a population of cells which includes dendritic cells or dendritic precursor cells that has been isolated from said individual to stimulate proliferation and maturation of dendritic cells;
b) contacting the dendritic cells with said scaffold molecule, wherein the antigens are expressed on the cancer cells;
c) removing CD25+/CD4+ cells from the population of cells; and
d) administering the population of cells without CD25+/CD4+ cells to the individual in an amount effective to stimulate a T cell response to the cancer cells.

12. The scaffold molecule of any of claims 1-6 for use in a method of treating an autoimmune disorder in an individual comprising:
a) culturing a population of cells which includes dendritic cells or dendritic precursor cells that has been isolated from said individual to stimulate proliferation and maturation of dendritic cells;
b) contacting the dendritic cells with said scaffold molecule, wherein the antigens are autoantigens;
c) collecting CD25+/CD4+ cells from the population of cells; and
d) administering the CD25+/CD4+ cells to the individual in an amount effective to suppress a symptom of the autoimmune disorder.

13. The scaffold molecule of any of claims 1 to 12 wherein the scaffold molecule comprises 15 or more different antigens.

14. A method to identify immunodominant antigen epitopes in a population of antigens comprising:
a) contacting a population of dendritic cells with scaffold molecules of any of claims 1-8:
b) collecting the population of cells;
c) eluting antigens from the surface of the population of cells; and
d) purifying the eluted antigens.
